# EUROPEAN PATENT SPECIFICATION

(11) **EP 2 950 754 B1**
(45) Date of publication and mention of the grant of the patent: **30.05.2018**
(21) Application number: 14745853.3
(22) Date of filing: 22.01.2014
(51) Int. Cl.: A61F 2/64, A61F 2/68, A61F 2/66, A61F 2/60, A61F 2/80

(54) **POLYCENTRIC PROSTHETIC KNEE**
POLYZENTRISCHE KNIEPROTHESE
PROTHÈSE DE GENOU POLYCENTRIQUE

(30) Priority: 01.02.2013 US 201361759978 P
(43) Date of publication of application: 09.12.2015
(73) Proprietor: LIMBS International Inc., El Paso TX 79901 (US)
(72) Inventor: TERLESKI, Timothy, W., Richardson, TX 75082 (US); GONZALEZ, Roger, V., El Paso, TX 79912 (US); CANTU, Cesar, Ernesto, El Paso, TX 79902 (US); LASALLE, Robert, K., Fort Worth, TX 76244 (US)
(74) Representative: Conroy, John
(86) International application number: PCT/US2014/012557
(87) International publication number: WO 2014/120532

(56) References cited:
- EP-A1- 2 432 430
- WO-A1-2011/123119
- US-A1- 2005 234 562
- US-A1- 2012 253 476
- US-B1- 8 268 012
- US-B2- 6 911 050
- US-B2- 8 231 688

## Description

### BACKGROUND

The following disclosure relates to polycentric prosthetic knees.

Some prosthetic knees have a single-axis joint mechanism that defines a fixed center of rotation throughout the bending motion of the knee. Other prosthetic knee joints have a multi-axis joint system in which the bending motion of the knee defines a non-stationary center of rotation. In a multi-axis knee joint, the instantaneous center of rotation moves with respect to the knee during the bending motion of the knee. Such polycentric joints provide advantages such as added stability and improved toe clearance during the swinging motion of the prosthesis. A device according to the preamble of claim 1 is known from EP2432430.

### SUMMARY

In a general aspect, a polycentric prosthetic knee includes multiple axles that collectively define the polycentric bending motion of the prosthetic knee.

In some aspects, a prosthetic knee includes an upper core knee member and a lower core knee member that rotates with respect to the upper core knee member about an instantaneous center of rotation during flexion of the prosthetic knee. The prosthetic knee includes axles that collectively define a location of the instantaneous center of rotation. The prosthetic knee includes one or more links connected between the upper core knee member and the lower core knee member.

The details of one or more implementations are set forth in the accompanying drawings and the description below. Other features, objects, and advantages will be apparent from the description and drawings, and from the claims.

### DESCRIPTION OF DRAWINGS

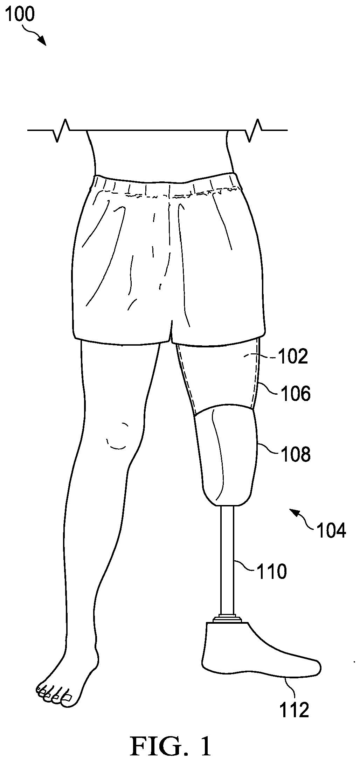

FIG. 1 is a diagram of an example trans-femoral amputee with a lower-limb prosthesis.

WO 2011/123119 describes a prosthetic knee that includes an upper member, a lower member, and two or more links. The upper member, the lower member, and the links may be made of non-metal material, including plastics, woods, and/or other types of material. The upper member carries a first upper axis and a second upper axis, and the lower member carries a first lower axis and a second lower axis. One or more of the links rotatably link the first upper axis and the first lower axis, and one or more of the links rotatably link the second upper axis and the second lower axis. The lower member can rotate with respect to the upper member about an instantaneous center of rotation defined by the first upper axis, the second upper axis, the first lower axis, and the second lower axis.

### SUMMARY

The invention is defined in the appended claims.

In a general aspect, a polycentric prosthetic knee includes multiple axles that collectively define the polycentric bending motion of the prosthetic knee.

In some aspects, a prosthetic knee includes an upper core knee member and a lower core knee member that rotates with respect to the upper core knee member about an instantaneous center of rotation during flexion of the prosthetic knee. The prosthetic knee includes axles that collectively define a location of the instantaneous center of rotation. The prosthetic knee includes one or more links connected between the upper core knee member and the lower core knee member.

The details of one or more implementations are set forth in the accompanying drawings and the description below. Other features, objects, and advantages will be apparent from the description and drawings, and from the claims.

### DESCRIPTION OF DRAWINGS

FIG. 1 is a diagram of an example trans-femoral amputee with a lower-limb prosthesis.
FIG. 2A is a perspective view of an example prosthetic knee 200 in an extended, locked configuration.
FIG. 2B is a top view of the example prosthetic knee 200 shown in FIG. 2A.
FIG. 2C is a bottom view of the example prosthetic knee 200 shown in FIG. 2A.
FIG. 2D is an anterior side view of the example prosthetic knee 200 shown in FIG. 2A.
FIG. 2E is a posterior side view of the example prosthetic knee 200 shown in FIG. 2A.
FIG. 2F is a right side view of the example prosthetic knee 200 shown in FIG. 2A.
FIG. 2G is a left side view of the example prosthetic knee 200 shown in FIG. 2A.
FIG. 2H is an anterior side view cross-section of the example prosthetic knee 200 shown in FIG. 2A.
FIG. 2I is another anterior side view cross-section of the example prosthetic knee 200 shown in FIG. 2A.
FIG. 2J is a left side view cross-section of the example prosthetic knee 200 shown in FIG. 2A.
FIG. 2K is a perspective view of the example prosthetic knee 200 without the knee cap 208.
FIG. 2L is an anterior side view of the example prosthetic knee 200 shown in FIG. 2K.
FIG. 2M is a left side view cross-section of the example prosthetic knee 200 shown in FIG. 2K.
FIG. 2N is a perspective view of the example prosthetic knee 200 in an extended, unlocked configuration.
FIG. 2O is an anterior side view of the example prosthetic knee 200 shown in FIG. 2K.
FIG. 2P is a left side view cross-section of the example prosthetic knee 200 shown in FIG. 2K.
FIG. 2Q is a perspective view of the example prosthetic knee 200 in a flexed, unlocked configuration.
FIG. 2R is an anterior side view of the example prosthetic knee 200 shown in FIG. 2Q.
FIG. 2S is a left side view cross-section of the example prosthetic knee 200 shown in FIG. 2Q.
FIG. 2T shows a perspective view of the example prosthetic knee 200 extended and having an elastic cord 230.
FIG. 2U is a bottom view of the example prosthetic knee 200 shown in FIG. 2T.
FIG. 2V is an anterior side view of the example prosthetic knee 200 shown in FIG. 2T.
FIG. 2W is a posterior side view of the example prosthetic knee 200 shown in FIG. 2T.
FIG. 2X is a left side view of the example prosthetic knee 200 shown in FIG. 2T.
FIG. 2Y is a left side view cross-section of the example prosthetic knee 200 shown in FIG. 2T.
FIG. 2Z shows a perspective view of the example prosthetic knee 200 flexed and having an elastic cord 230, and having a locked extension assist tensioner system.

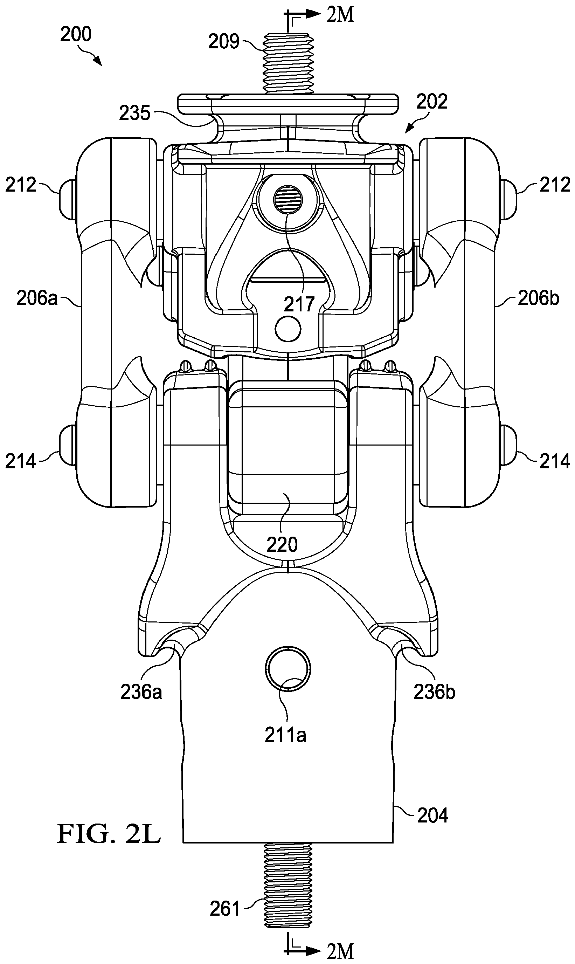

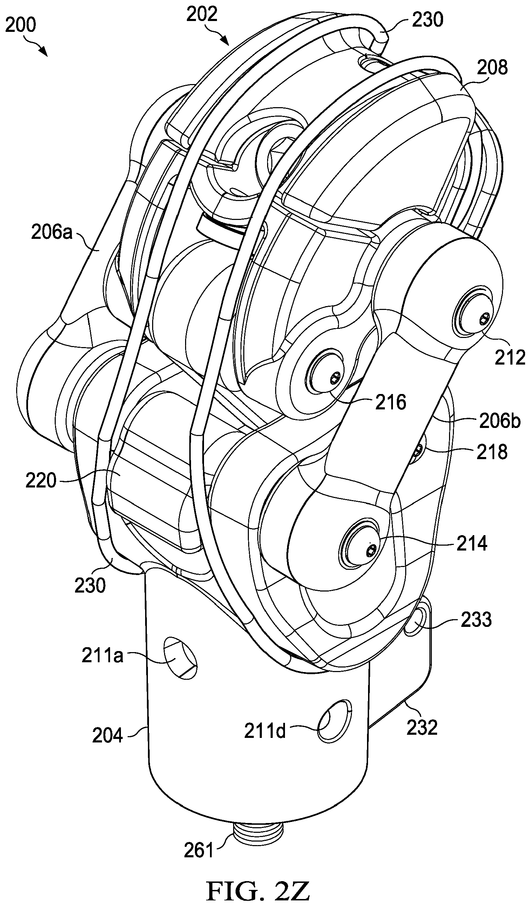

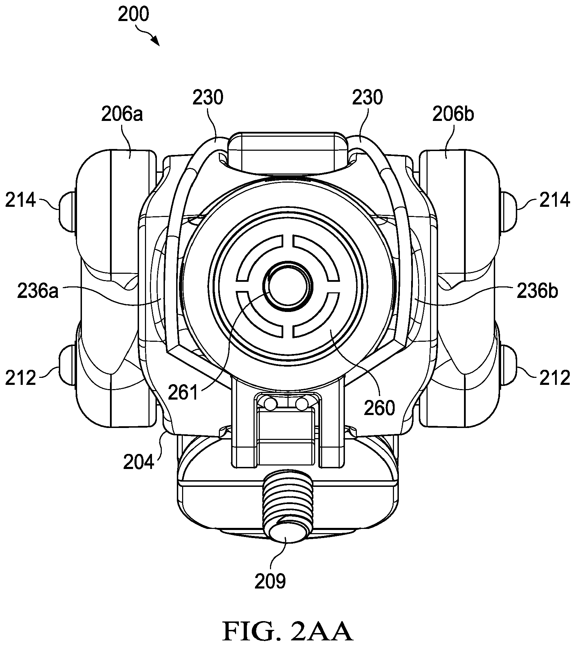

FIG. 2AA is a bottom view of the example prosthetic knee 200 shown in FIG. 2Z.

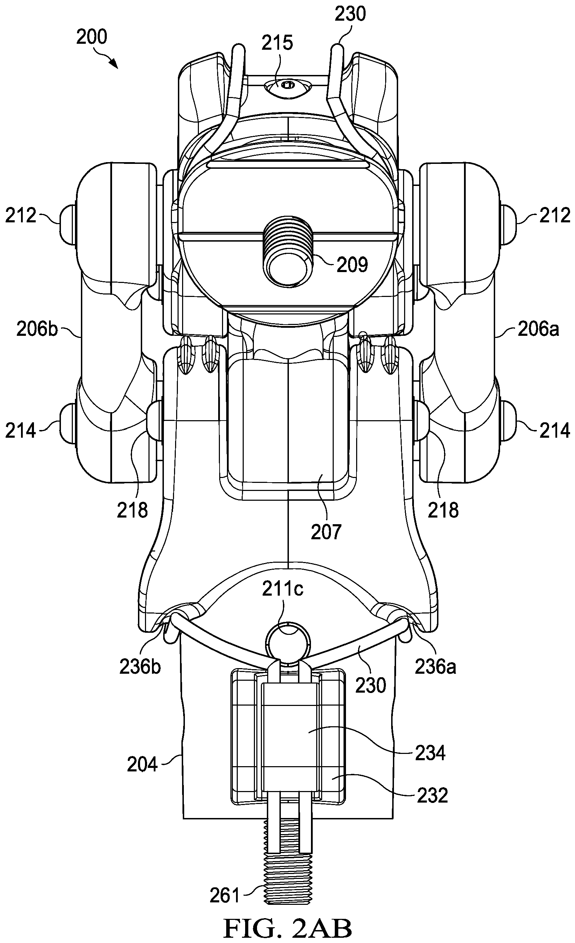

FIG. 2AB is a posterior side view of the example prosthetic knee 200 shown in FIG. 2Z.

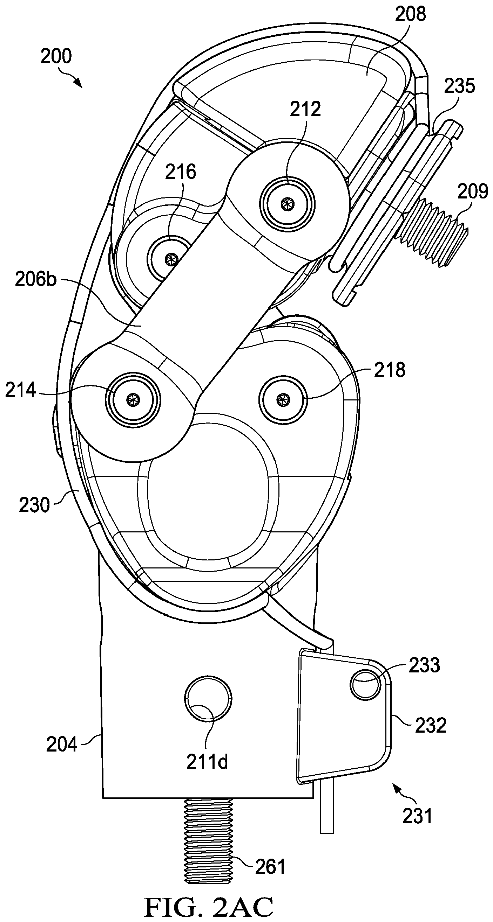

FIG. 2AC is a left side view of the example prosthetic knee 200 shown in FIG. 2Z.

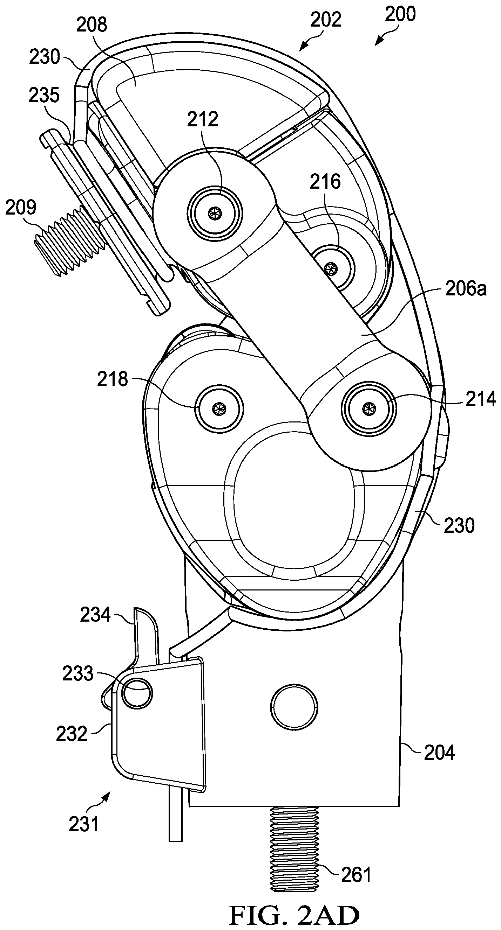

FIG. 2AD is a right side view of the example prosthetic knee 200 flexed, and having an unlocked extension assist tensioner system 231.

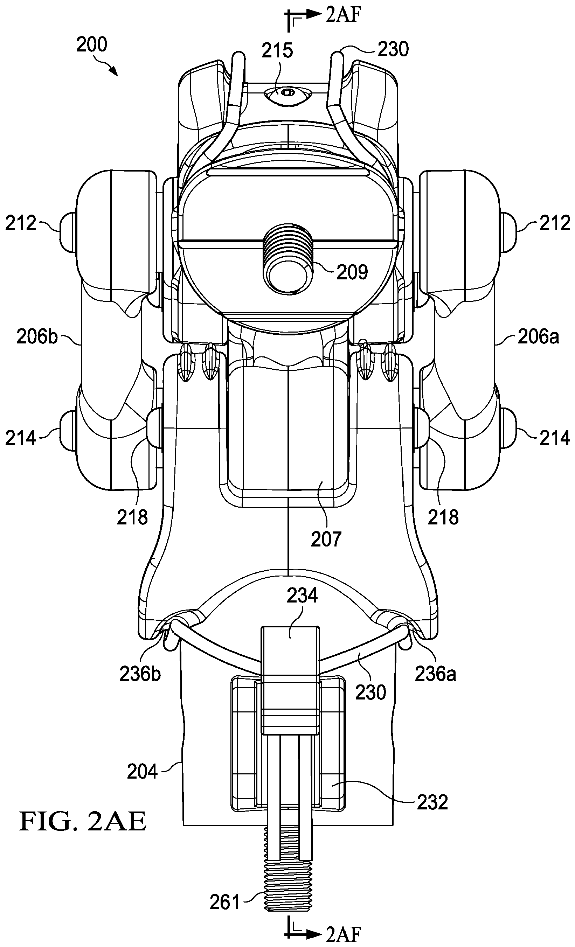

FIG. 2AE is a posterior side view of the example prosthetic knee 200 shown in FIG. 2AD.

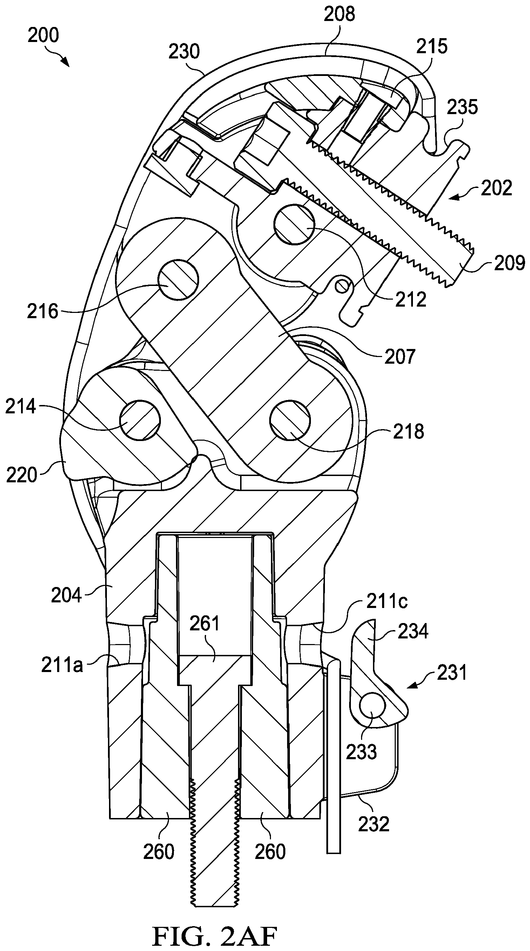

FIG. 2AF is a left side view cross-section of the example prosthetic knee 200 shown in FIG. 2AD.

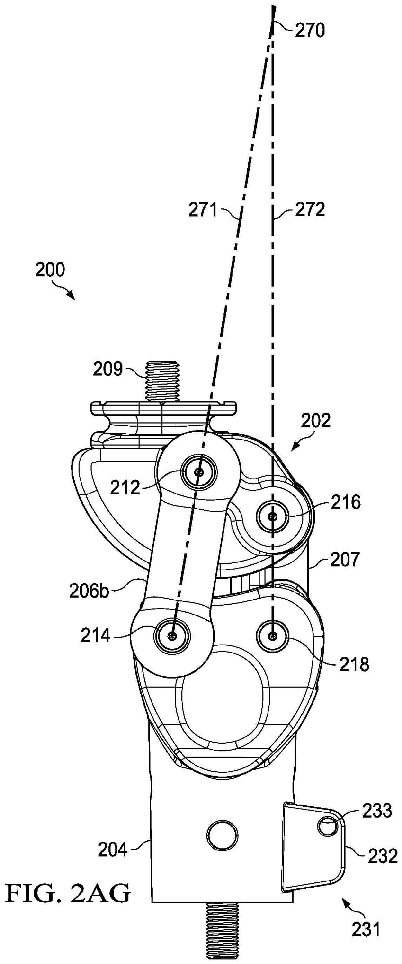

FIG. 2AG is a left side view of the example prosthetic knee 200 in an extended configuration, showing the instantaneous center of rotation 270.

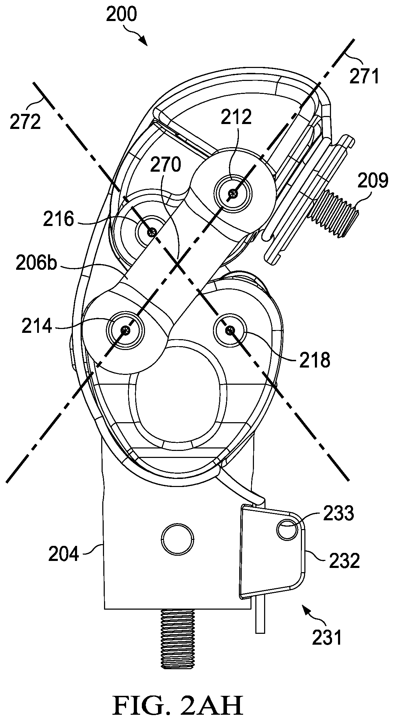

FIG. 2AH is a left side view of the example prosthetic knee 200 in an flexed configuration, showing the instantaneous center of rotation 270.

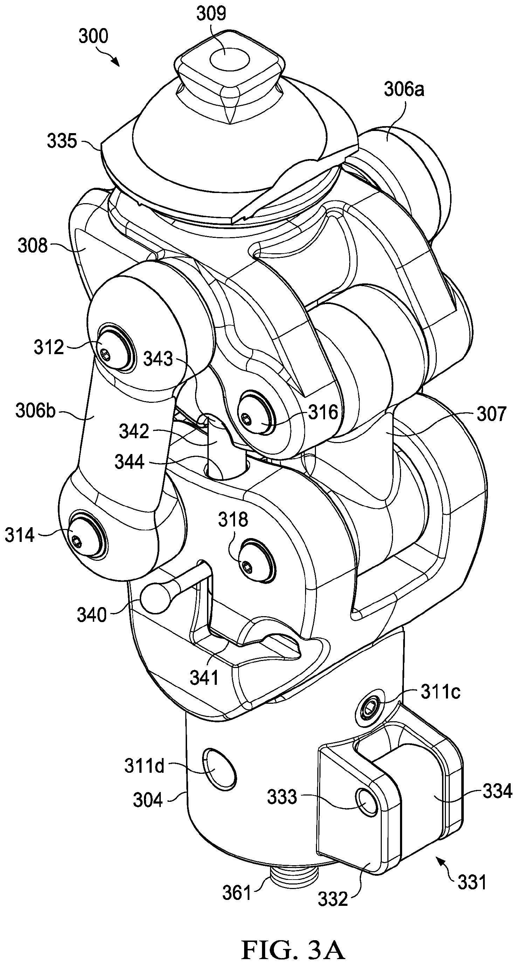

FIG. 3A is a perspective view of an example prosthetic knee 300 in an extended, locked configuration.

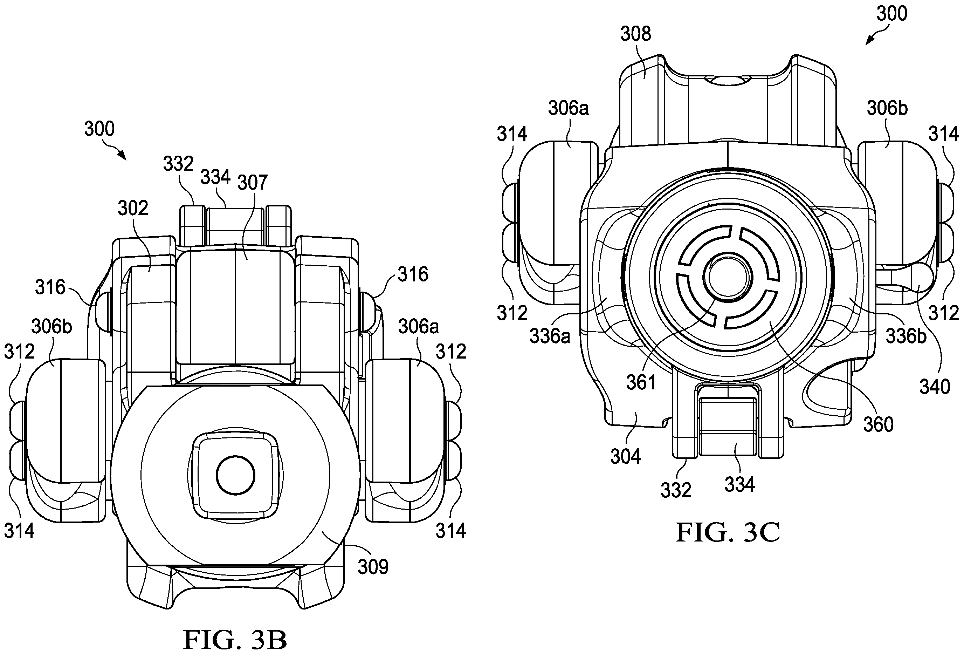

FIG. 3B is a top view of the example prosthetic knee 300 shown in FIG. 3A.
FIG. 3C is a bottom view of the example prosthetic knee 300 shown in FIG. 3A.

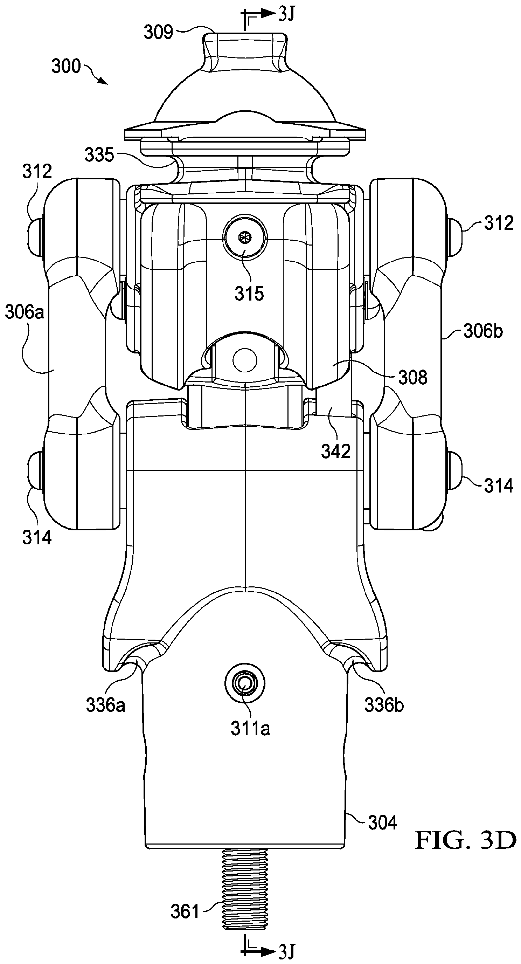

FIG. 3D is an anterior side view of the example prosthetic knee 300 shown in FIG. 3A.

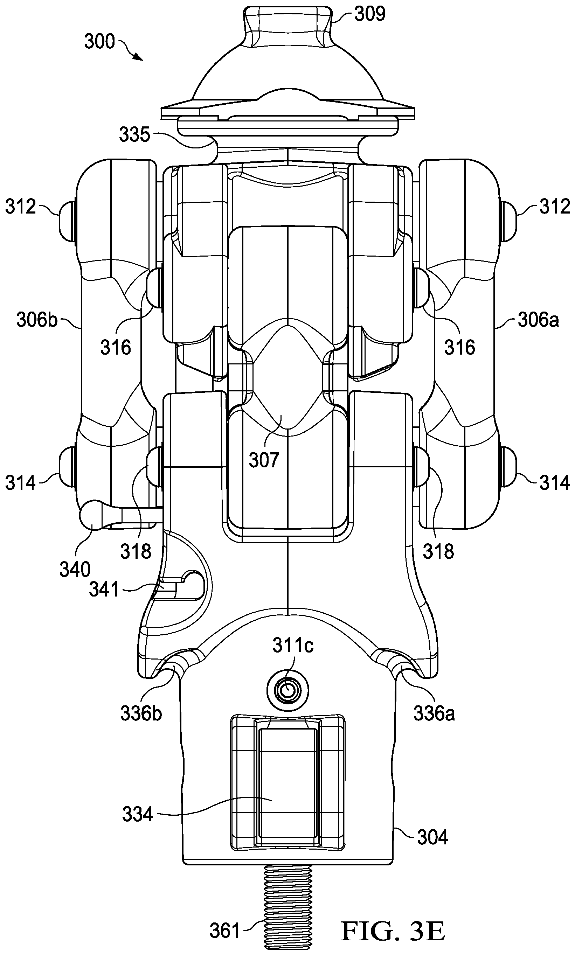

FIG. 3E is a posterior side view of the example prosthetic knee 300 shown in FIG. 3A.

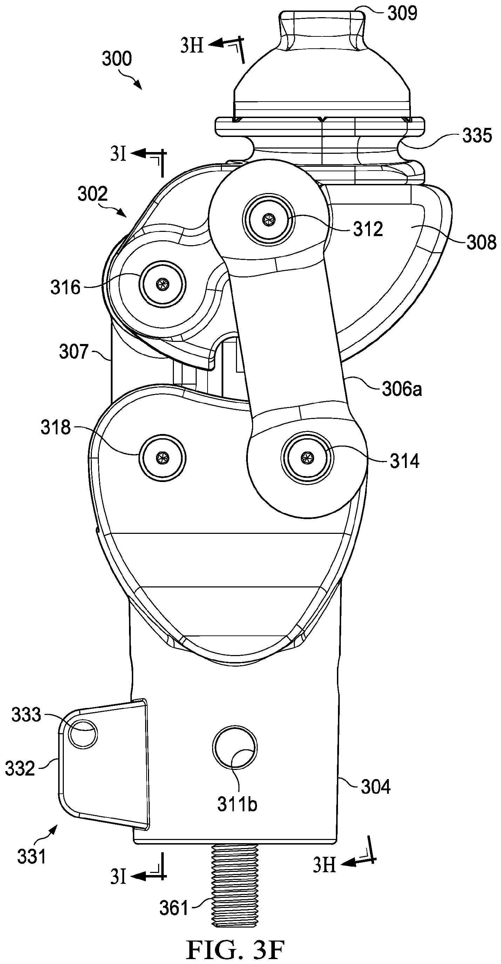

FIG. 3F is a right side view of the example prosthetic knee 300 shown in FIG. 3A.

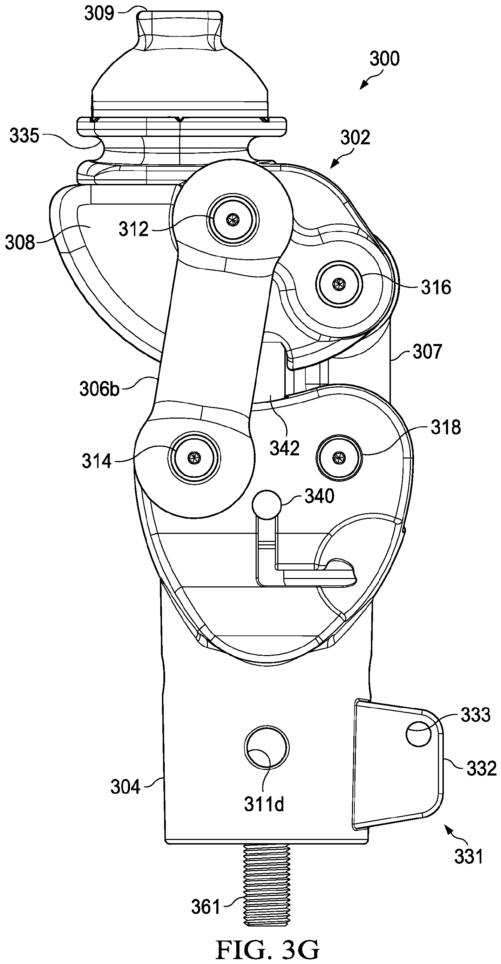

FIG. 3G is a left side view of the example prosthetic knee 300 shown in FIG. 3A.

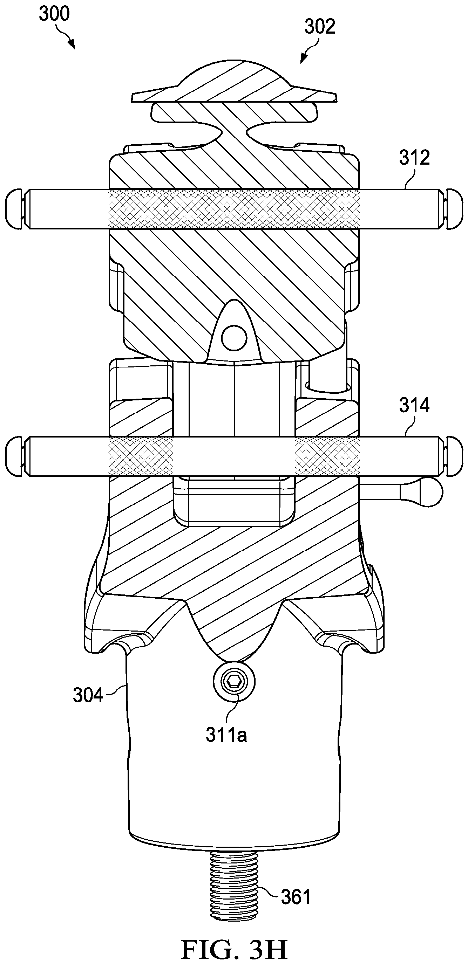

FIG. 3H is an anterior side view cross-section of the example prosthetic knee 300 shown in FIG. 3A.

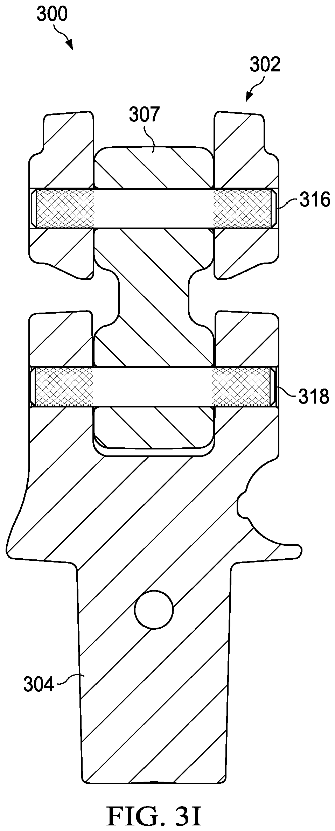

FIG. 3I is another anterior side view cross-section of the example prosthetic knee 300 shown in FIG. 3A.

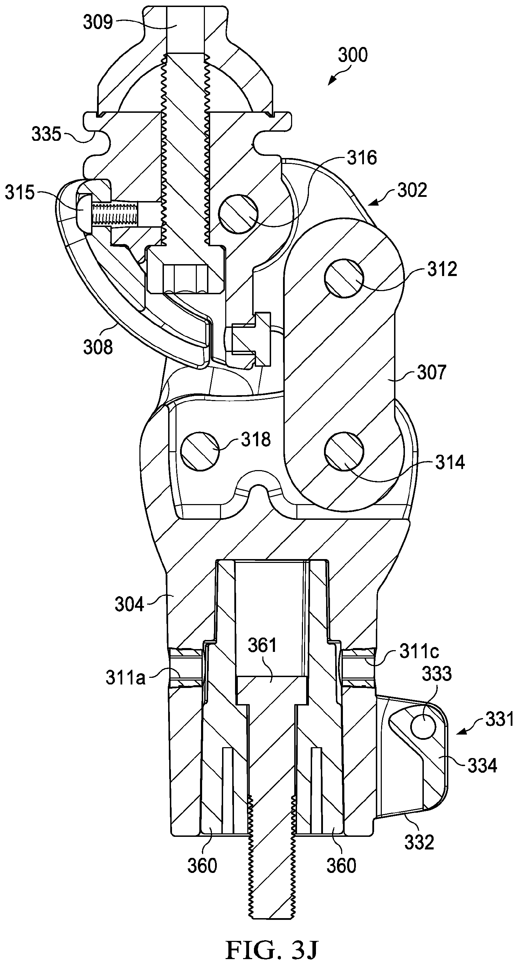

FIG. 3J is a left side view cross-section of the example prosthetic knee 300 shown in FIG. 3A.

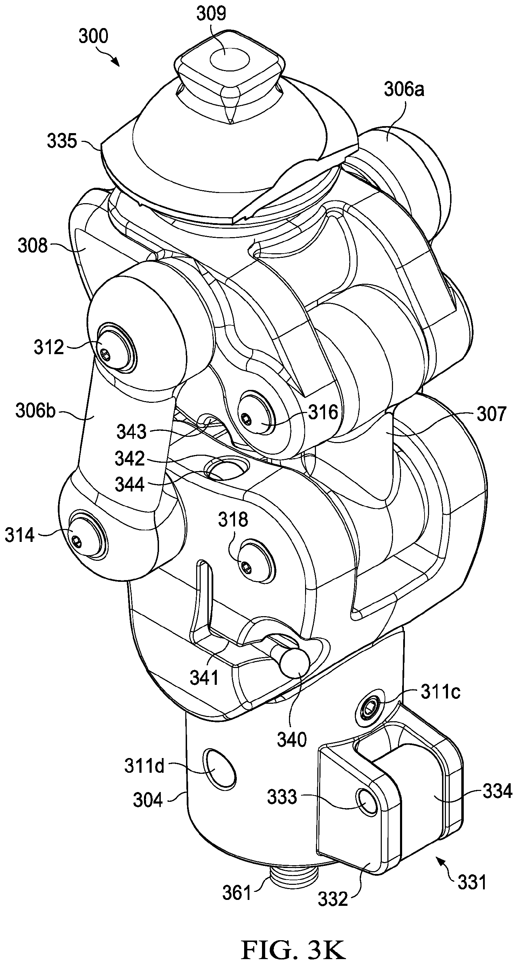

FIG. 3K is a perspective view of an example prosthetic knee 300 in an extended, unlocked configuration.

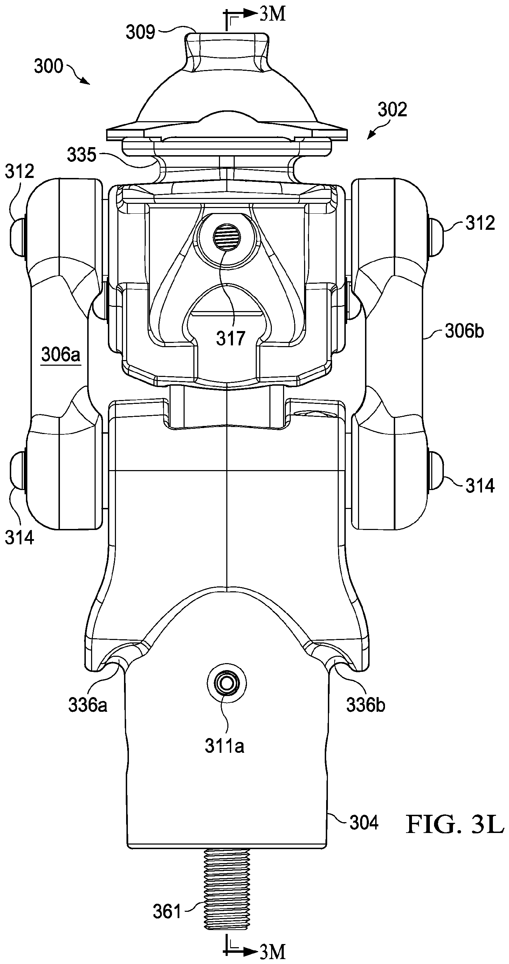

FIG. 3L is an anterior side view of the example prosthetic knee 300 shown in FIG. 3K without the knee cap 308.

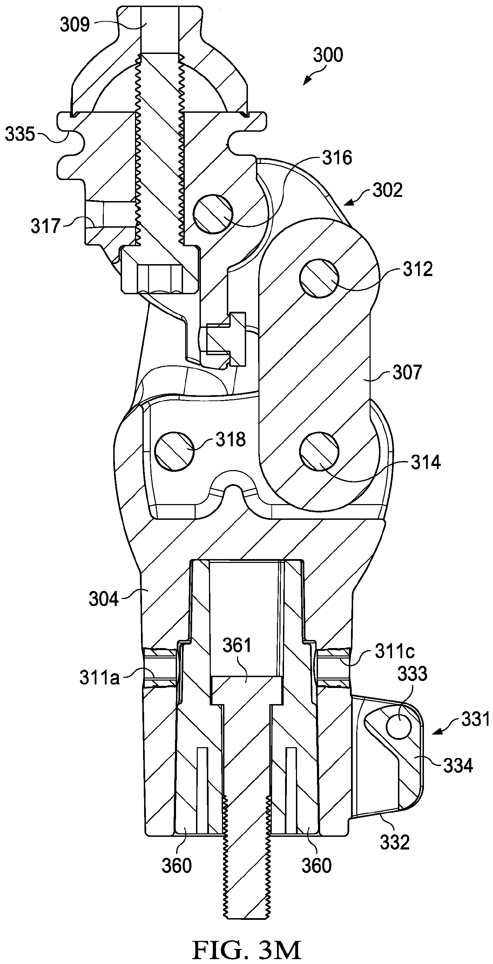

FIG. 3M is a left side view cross-section of the example prosthetic knee 300 shown in FIG. 3L.

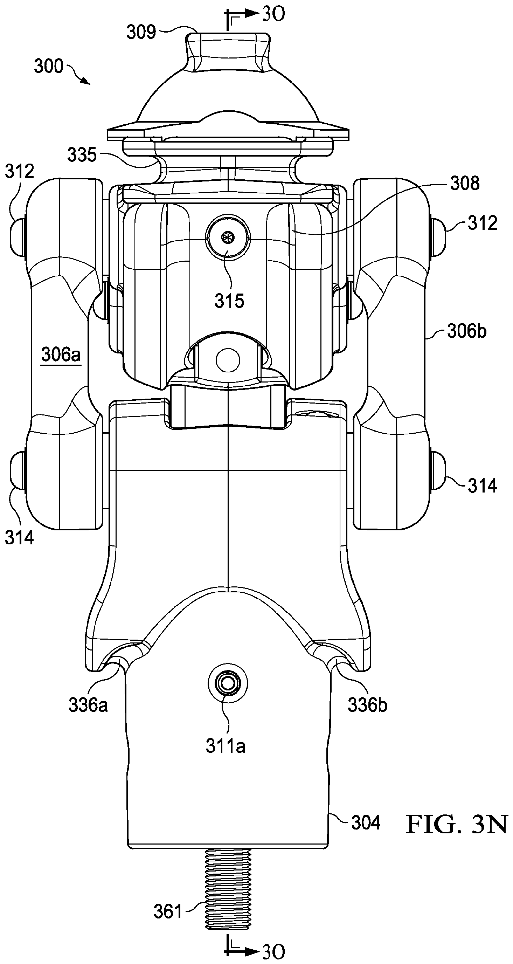

FIG. 3N is an anterior side view of the example prosthetic knee 300 in an extended, unlocked configuration with the knee cap 308.

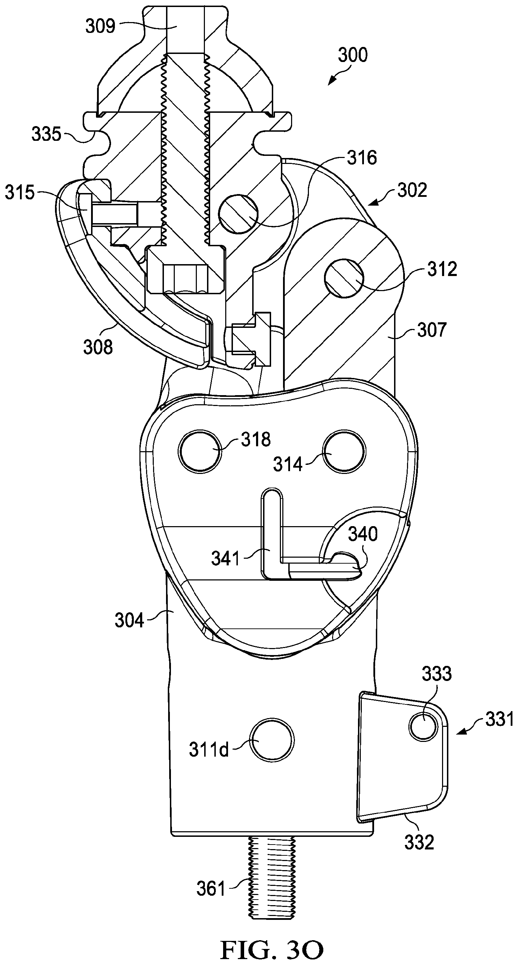

FIG. 3O is a left side view cross-section of the example prosthetic knee 300 shown in FIG. 3N.

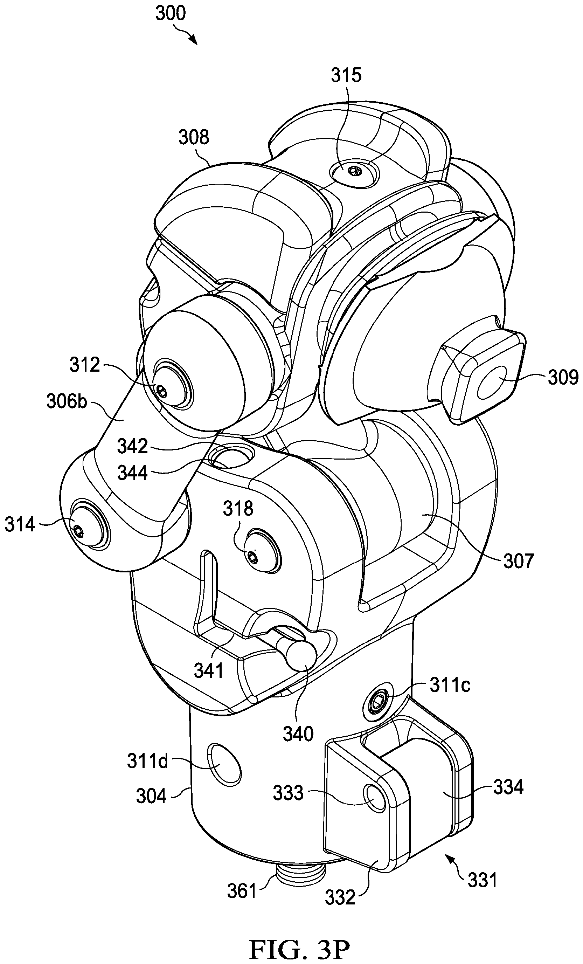

FIG. 3P is a perspective view of the example prosthetic knee 300 in a flexed, unlocked configuration.

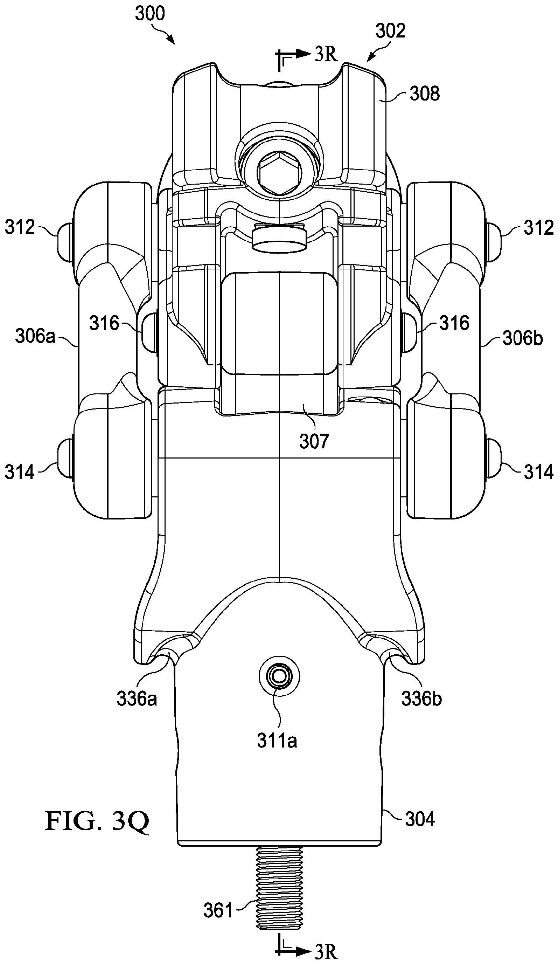

FIG. 3Q is an anterior side view of the example prosthetic knee 300 shown in FIG. 3P.

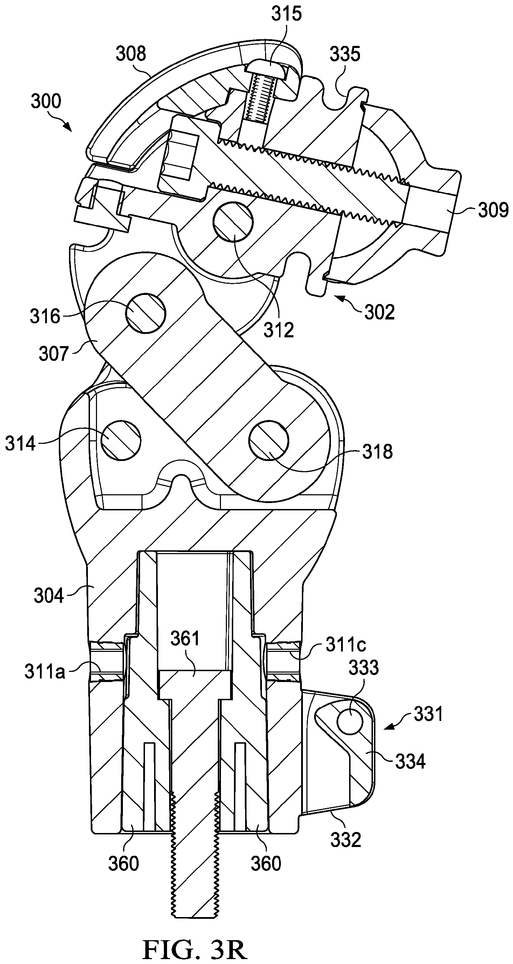

FIG. 3R is a left side view cross-section of the example prosthetic knee 300 shown in FIG. 3P.

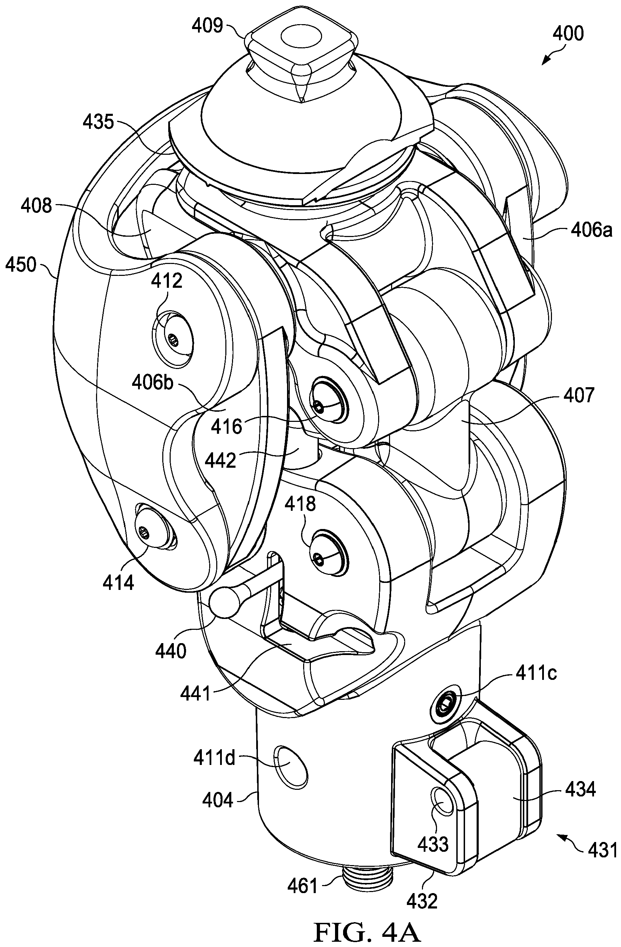

FIG. 4A is a perspective view of an example prosthetic knee 400 in an extended, locked configuration.

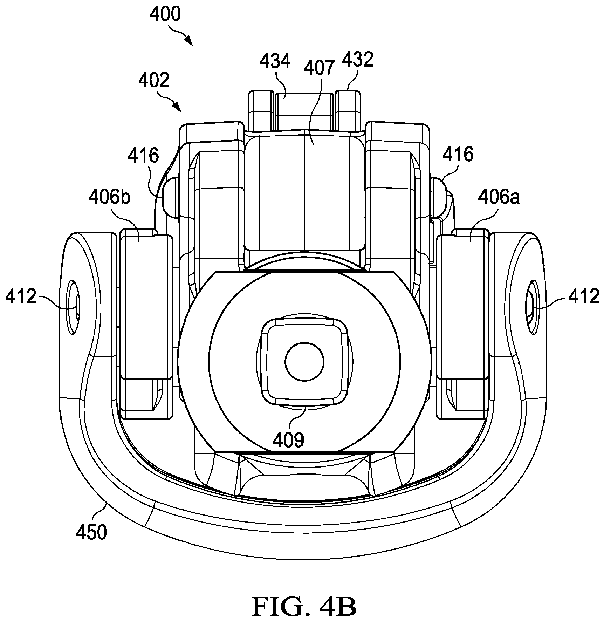

FIG. 4B is a top view of the example prosthetic knee 400 shown in FIG. 4A.

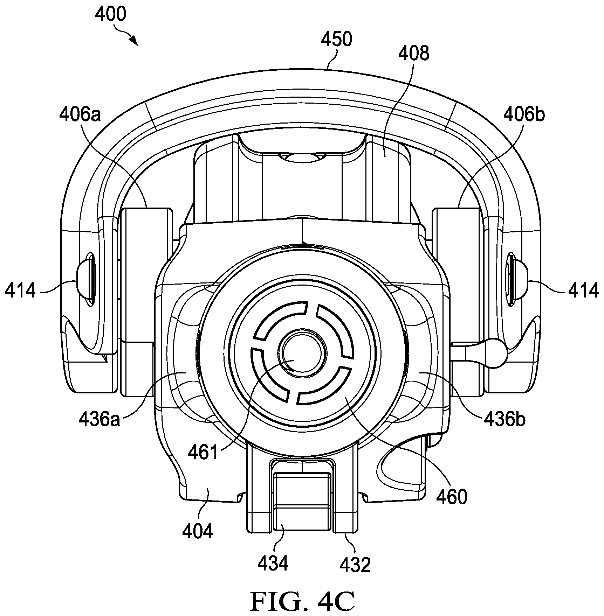

FIG. 4C is a bottom view of the example prosthetic knee 400 shown in FIG. 4A.

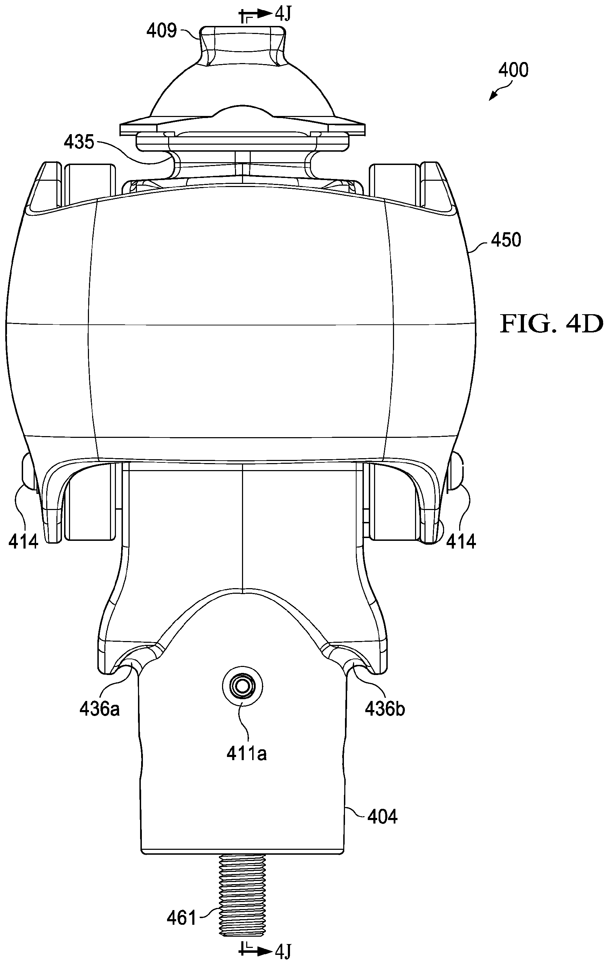

FIG. 4D is an anterior side view of the example prosthetic knee 400 shown in FIG. 4A.

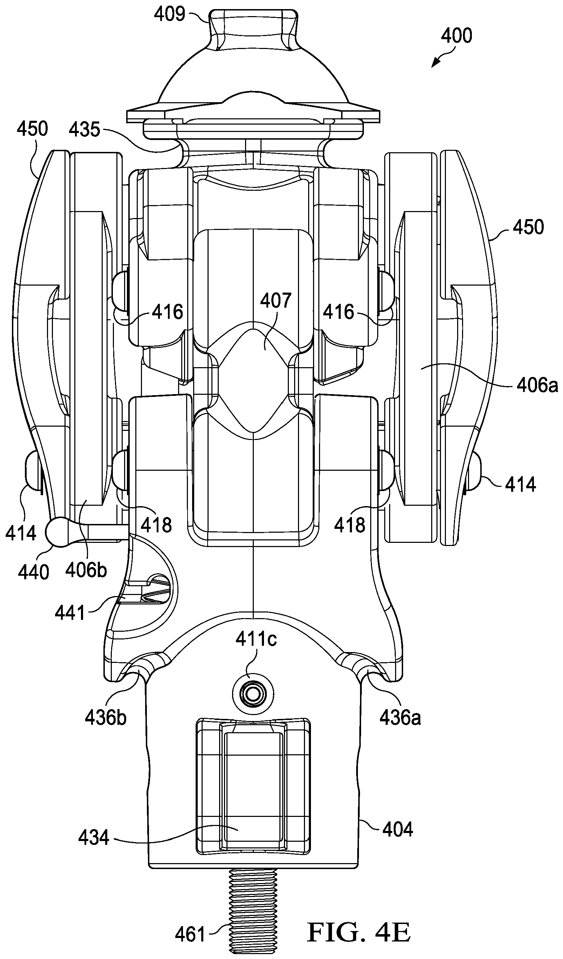

FIG. 4E is a posterior side view of the example prosthetic knee 400 shown in FIG. 4A.

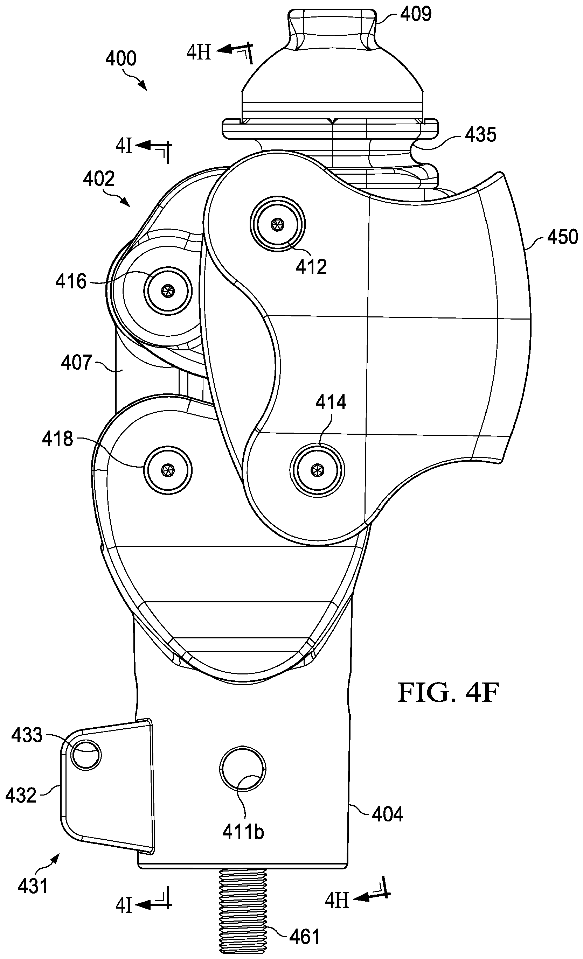

FIG. 4F is a right side view of the example prosthetic knee 400 shown in FIG. 4A.

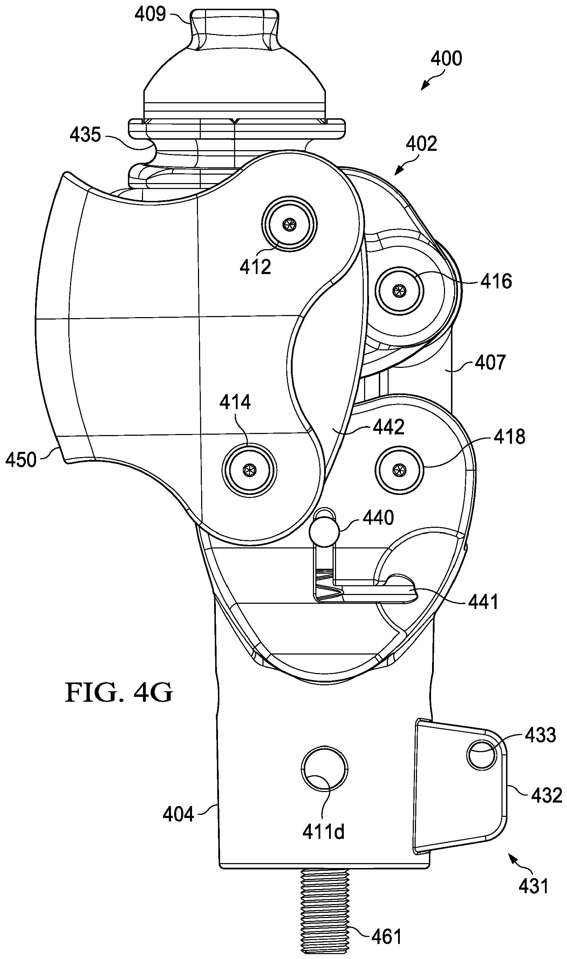

FIG. 4G is a left side view of the example prosthetic knee 400 shown in FIG. 4A.

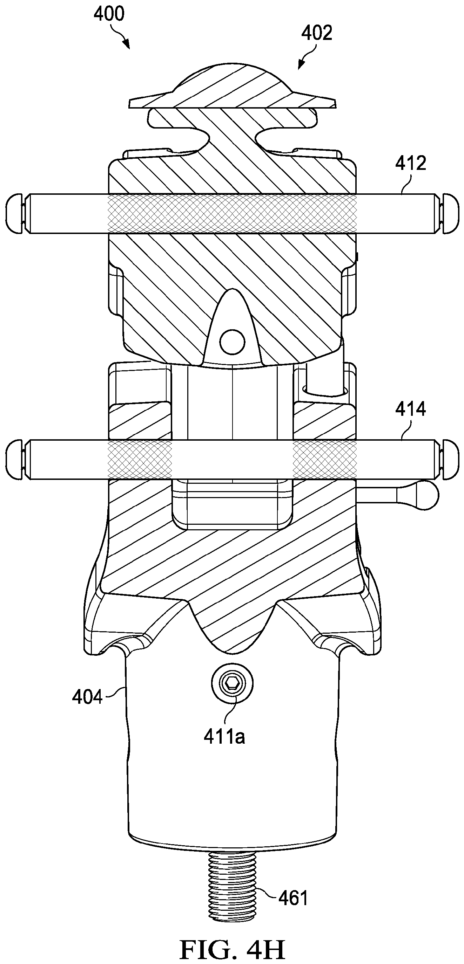

FIG. 4H is an anterior side view cross-section of the example prosthetic knee 400 shown in FIG. 4A.

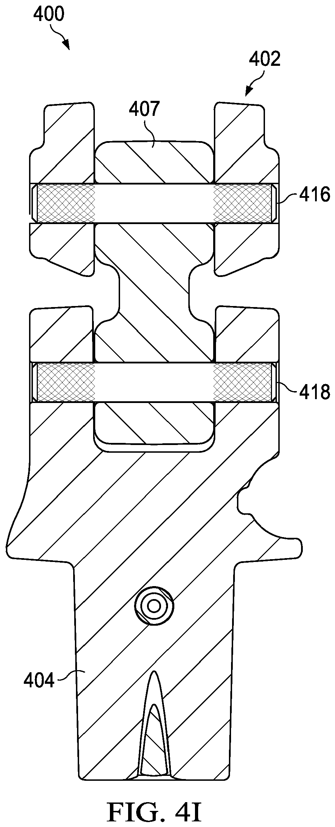

FIG. 4I is another anterior side view cross-section of the example prosthetic knee 400 shown in FIG. 4A.

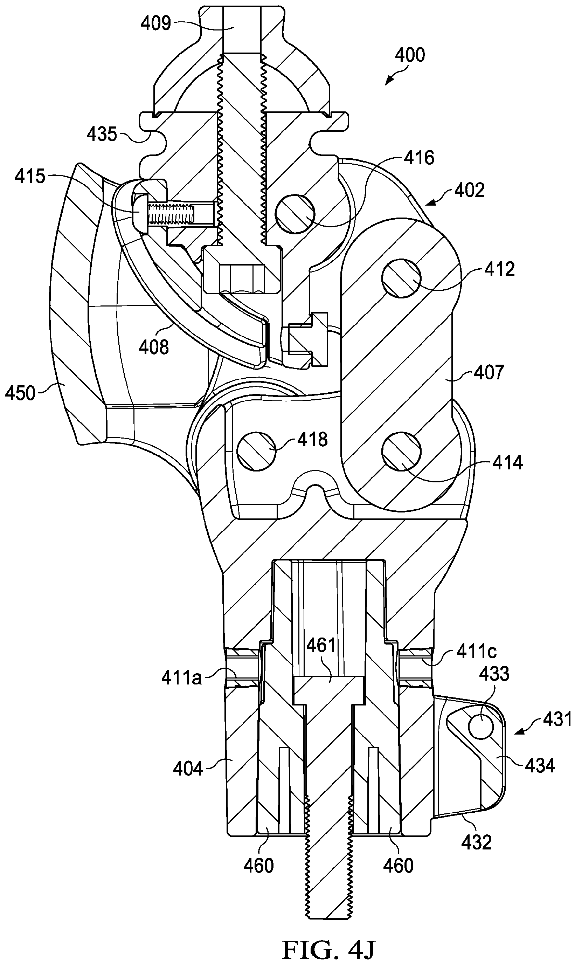

FIG. 4J is a left side view cross-section of the example prosthetic knee 400 shown in FIG. 4A.

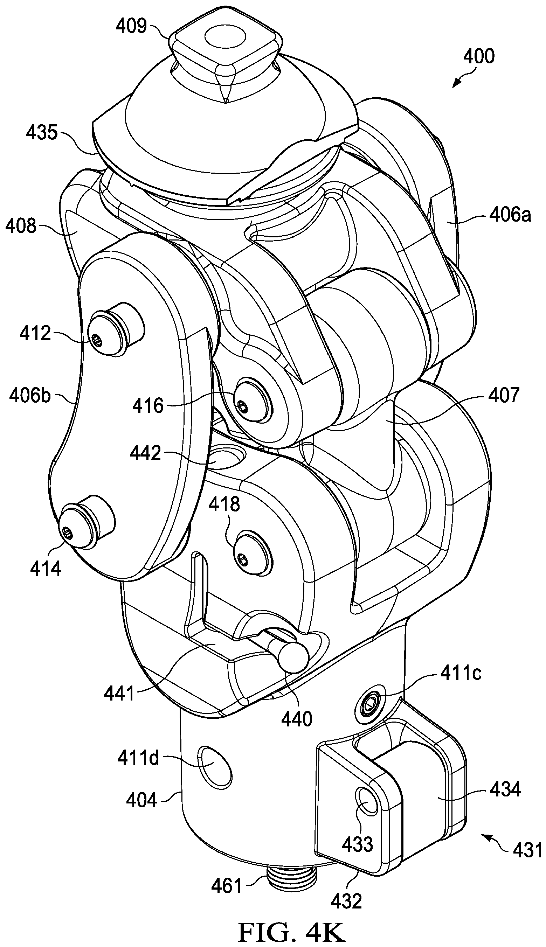

FIG. 4K is a perspective view of the example prosthetic knee 400 without the knee cover 450.

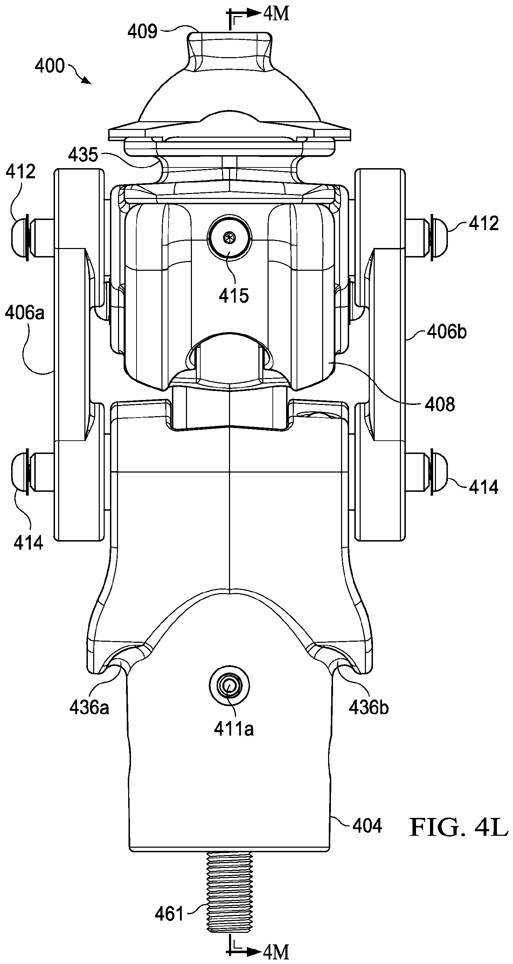

FIG. 4L is an anterior side view of the example prosthetic knee 400 shown in FIG. 4K.

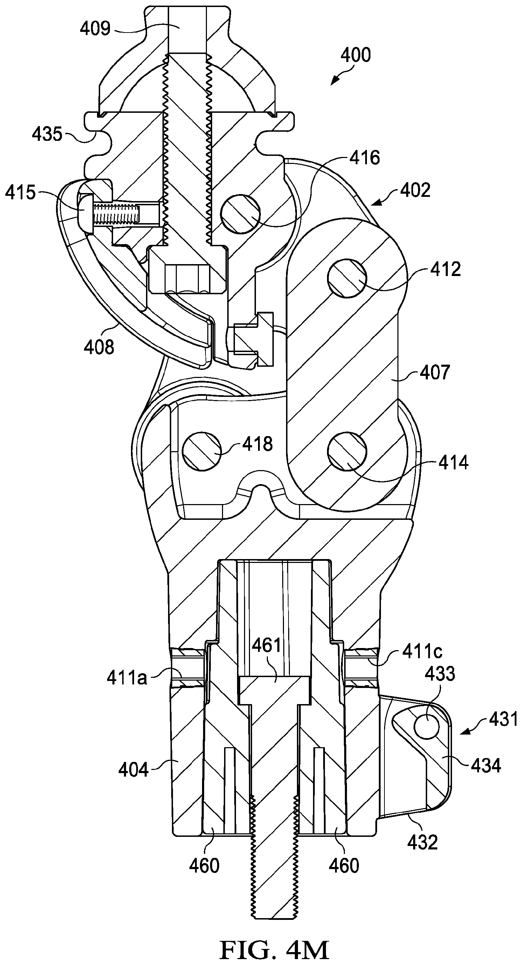

FIG. 4M is a left side view cross-section of the example prosthetic knee 400 shown in FIG. 4K.

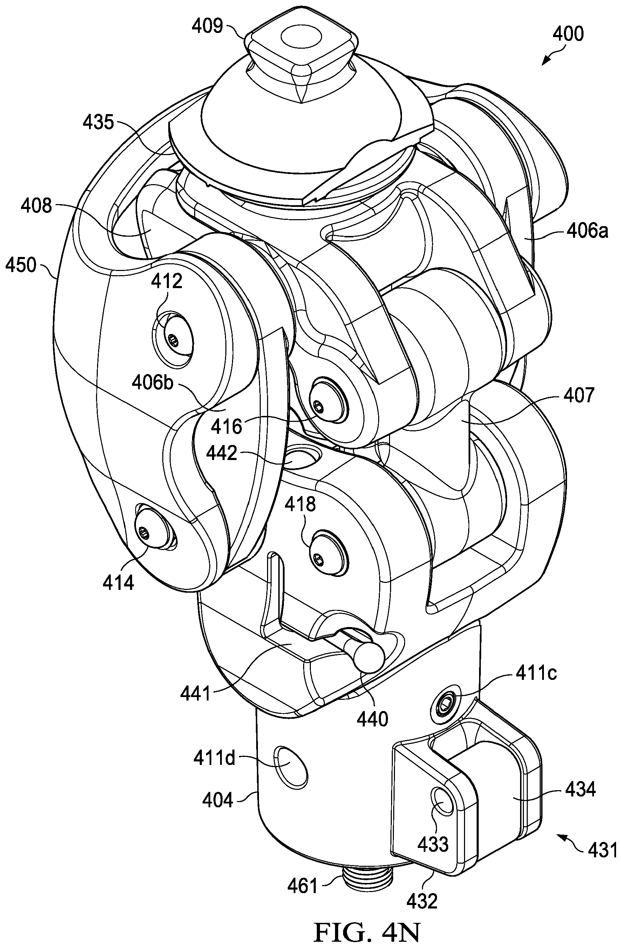

FIG. 4N is a perspective view of the example prosthetic knee 400 in an extended, unlocked configuration.

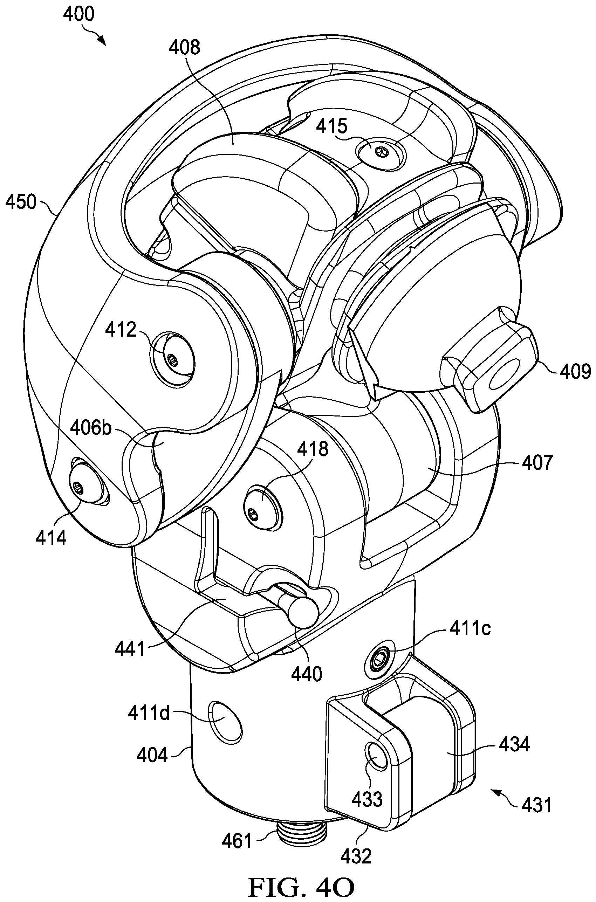

FIG. 4O is a perspective view of the example prosthetic knee 400 in a flexed, unlocked configuration.

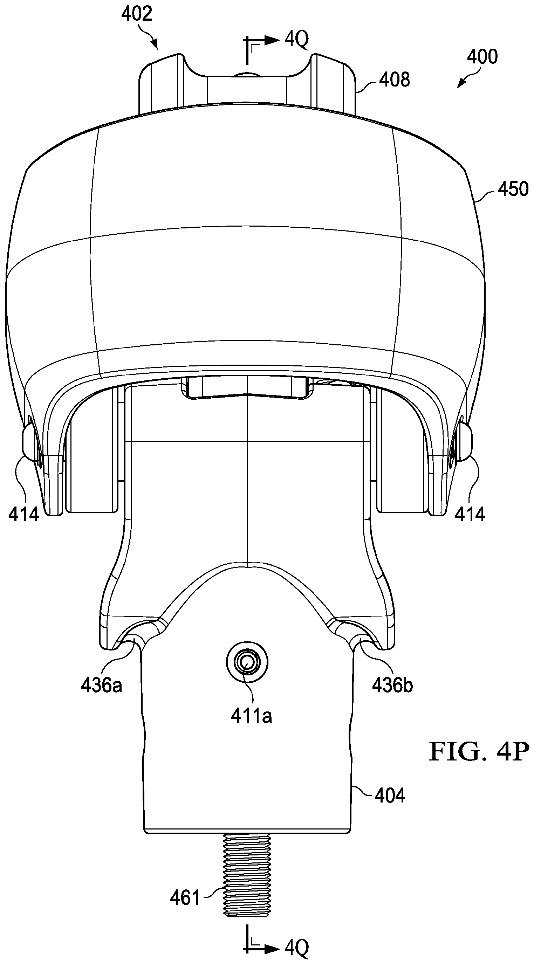

FIG. 4P is an anterior side view of the example prosthetic knee 400 shown in FIG. 4O.

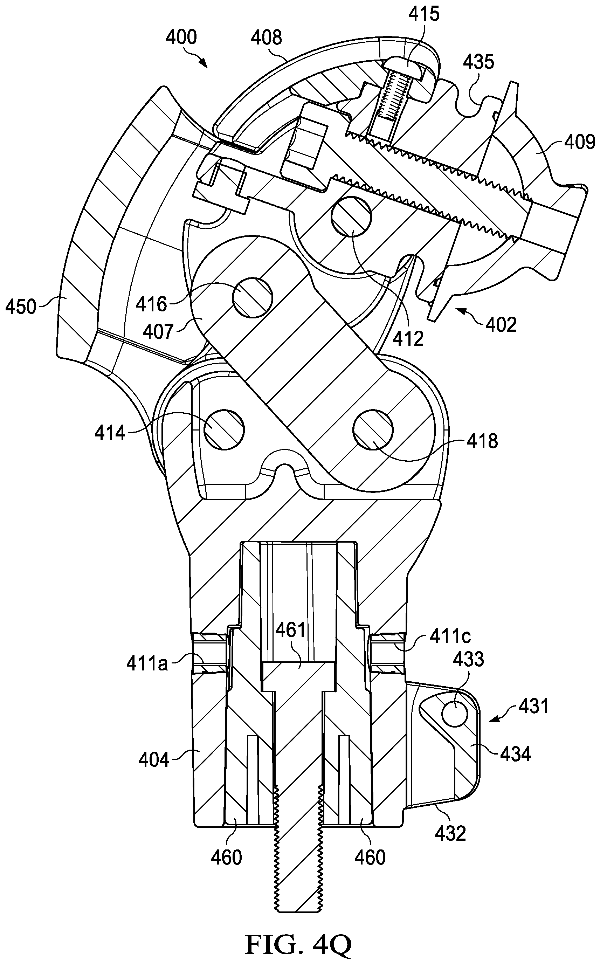

FIG. 4Q is a left side view cross-section of the example prosthetic knee 400 shown in FIG. 4O.

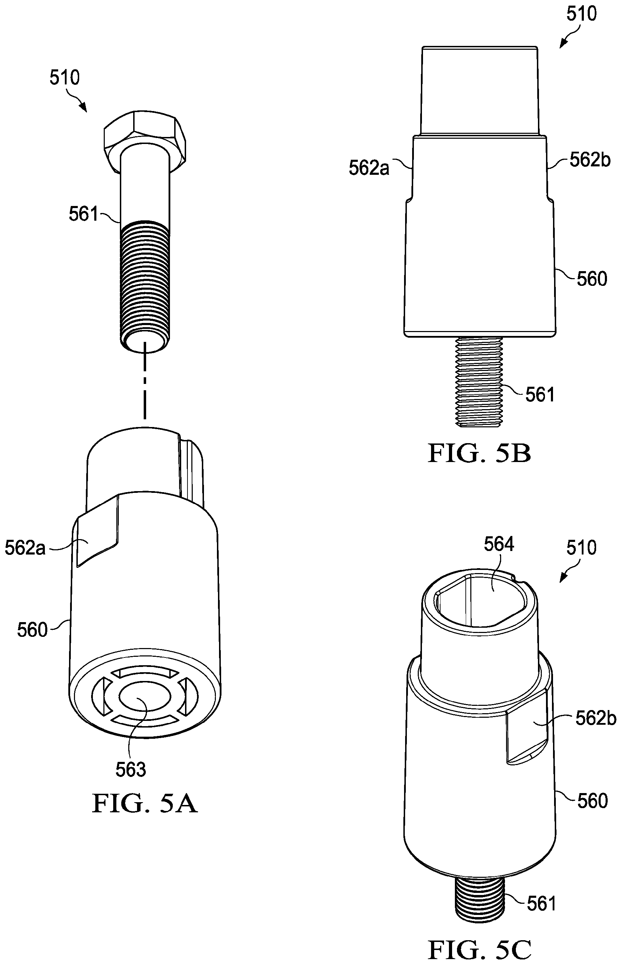

FIG. 5A is a perspective view of an example pylon adapter insert 510.
FIG. 5B is a side view of the example pylon adapter insert 510.
FIG. 5C is another perspective view of the example pylon adapter insert 510.

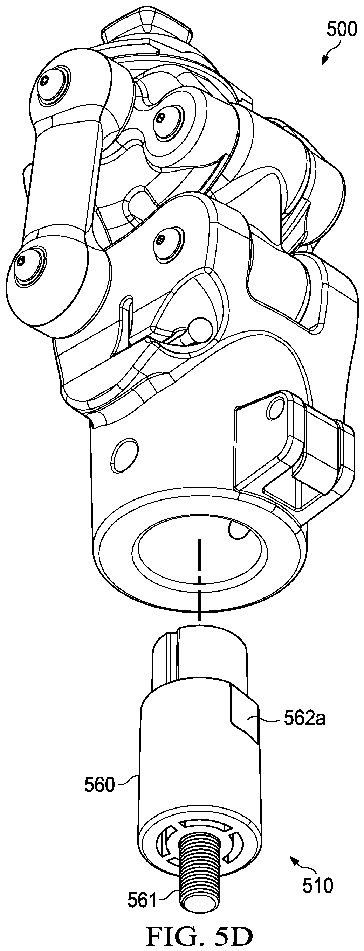

FIG. 5D is a perspective view of the pylon adapter insert 510 outside the lower bore of the lower core member of an example prosthetic knee.

Like reference symbols in the various drawings indicate like elements.

### DETAILED DESCRIPTION

FIG. 1 is a diagram of an example transfemoral amputee 100 having a residual limb 102 that carries a lower-limb prosthesis 104. The example transfemoral amputee 100 is a human whose left leg has been amputated above the knee, resulting in the residual limb 102 that includes a portion of the amputee's thigh. Generally, a transfemoral amputee may have an amputated left leg, an amputated right leg, or both. As such, the lower-limb prosthesis 104 may be secured to a left residual limb or a right residual limb, or an amputee having both legs amputated may carry a prosthesis on each of the right and left residual limbs.

The example prosthesis 104 shown includes a socket 106, a prosthetic knee 108, a pylon 110, and a prosthetic foot 112. A prosthesis may include additional or different features in the configuration shown or in a different configuration. The socket 106 secures to the residual limb, and the example prosthetic knee 108 secures to the socket. The example prosthetic knee 108 provides a bending motion of the prosthesis 104. Additional or different bending or flexing motion may be provided by other components of the prosthesis 104, for example, the prosthetic foot 112, a prosthetic ankle, or others. The example prosthetic knee 108 has an extended position corresponding to an extended position of the prosthesis 104. When the prosthesis 104 is in its extended position, the pylon 110 is substantially aligned with the residual limb 102. The prosthesis 104 in its extended position can support the weight of the amputee 100.

The example prosthetic knee 108 has a range of flexed positions corresponding to a range of the bending motion of the prosthesis 104. The range of motion corresponds to a range of angles between the pylon 110 and the residual limb 102. For example, the prosthesis 104 can traverse at least a portion of the range motion during the swing phase of the amputee's walking motion. Flexion of the example prosthetic knee 108 may allow the prosthesis 104 to accommodate the amputee 100 sitting, kneeling, standing, walking, jogging, swimming, or performing any combination of these and other functions.

The example prosthetic knee 108 can be an external, polycentric, four-bar joint design, or the prosthetic knee 108 can have another design. The example prosthetic knee 108 can be external, for example, where the joint is designed for external application to a residual limb, rather than internal (e.g., surgical) implantation within the body. A four-bar joint design can include four mechanical axes that prescribe the bending motion of the knee. For example, a prosthetic knee can have four axes of mechanical rotation: an upper posterior axis, a lower posterior axis, an upper anterior axis, and a lower anterior axis. A polycentric joint can have an instantaneous center of rotation that moves with respect to the mechanical axes during at least part of the bending motion of the knee. The instantaneous center of rotation can be defined, for example, by the upper posterior axis, the lower posterior axis, the upper anterior axis, and the lower anterior axis. In some instances, a polycentric knee design may provide the prosthesis 104 with improved ground clearance during the swing phase, a more stable extended position, or other advantages.

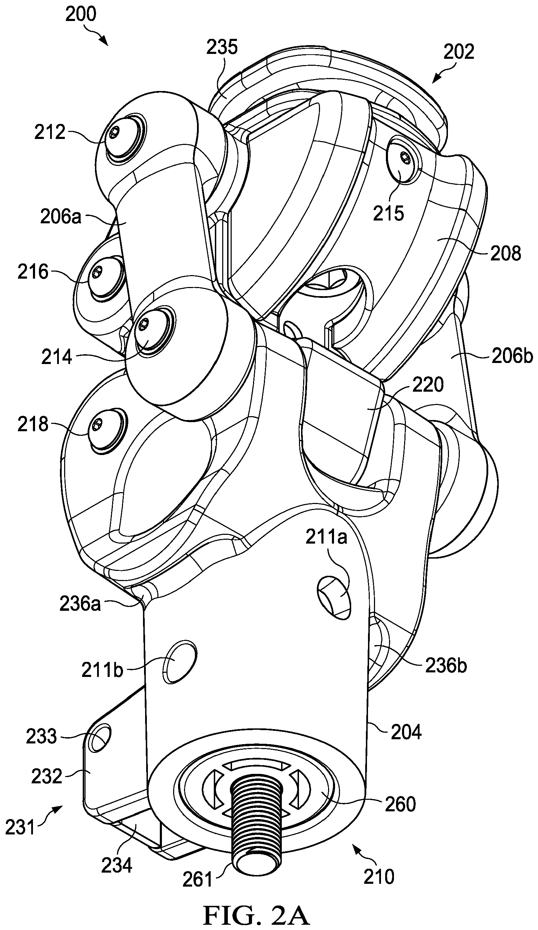

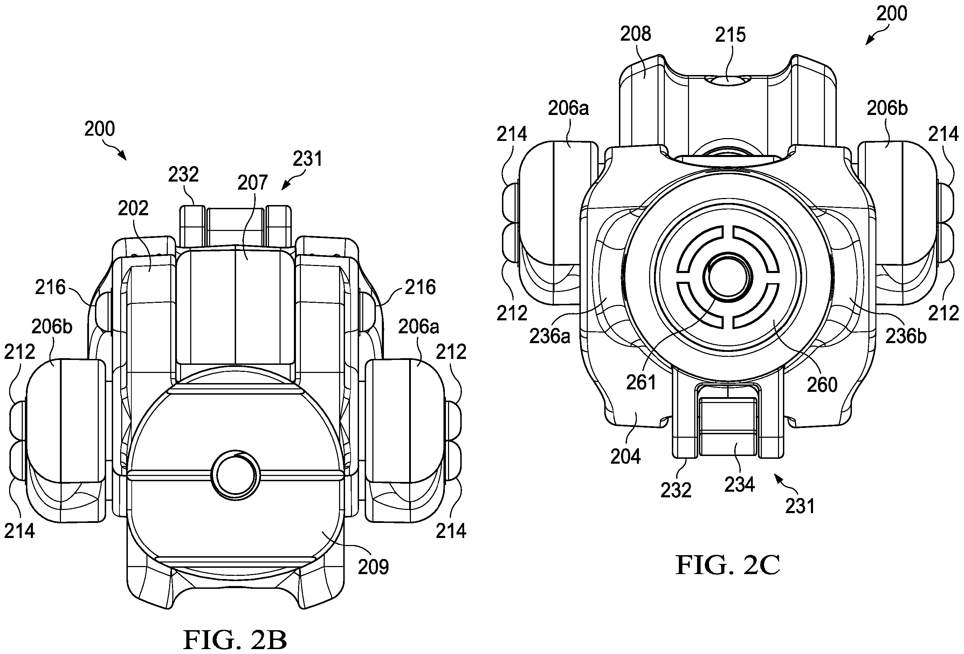

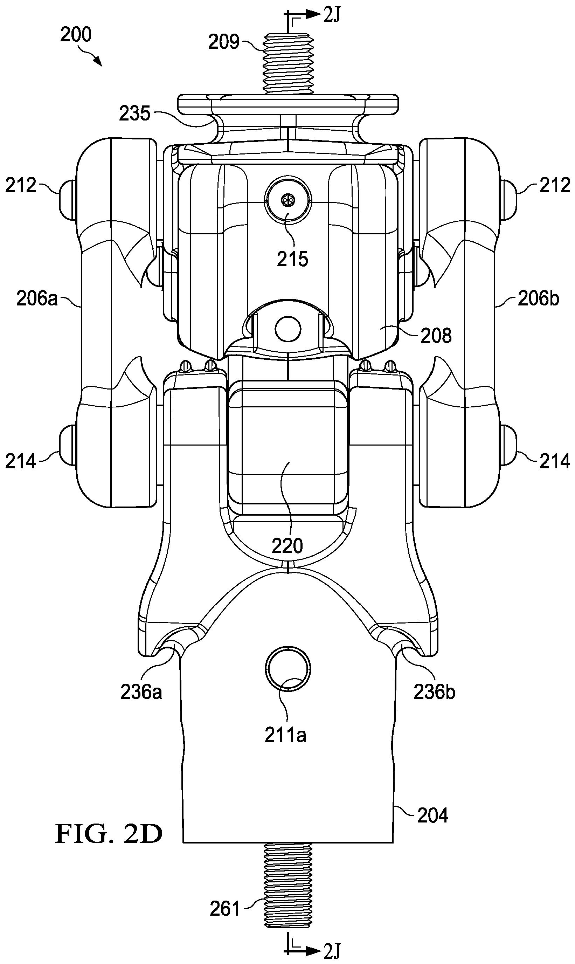

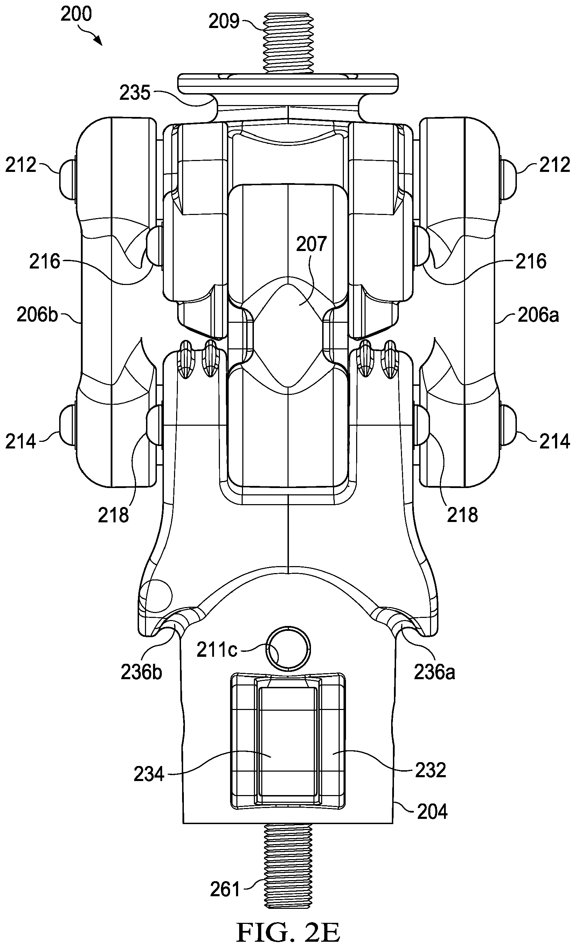

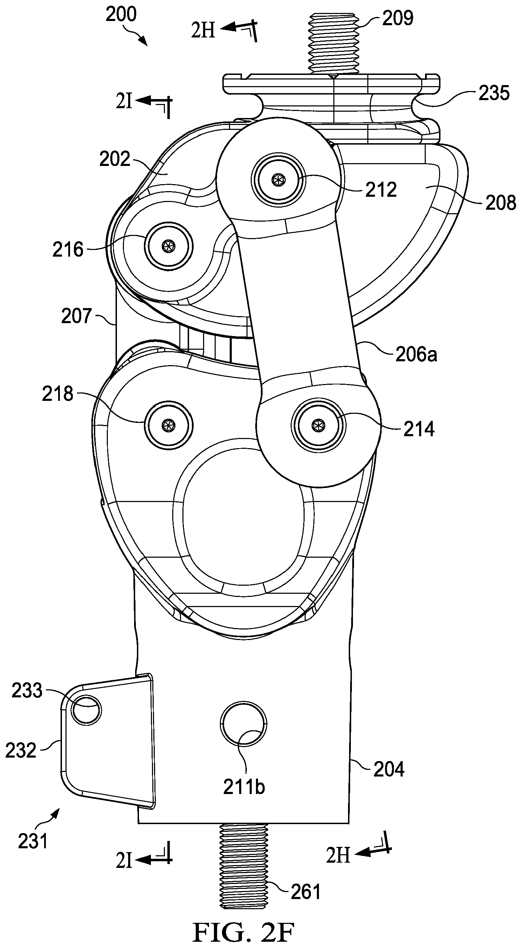

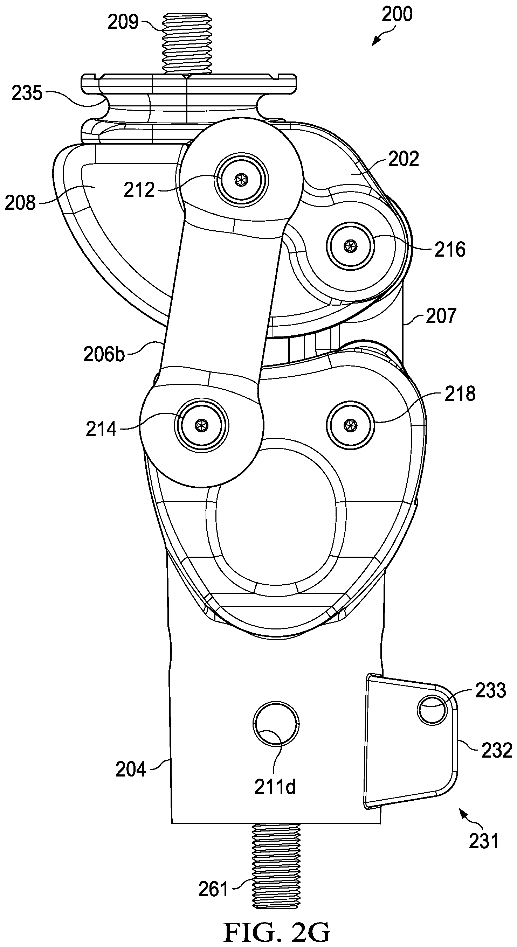

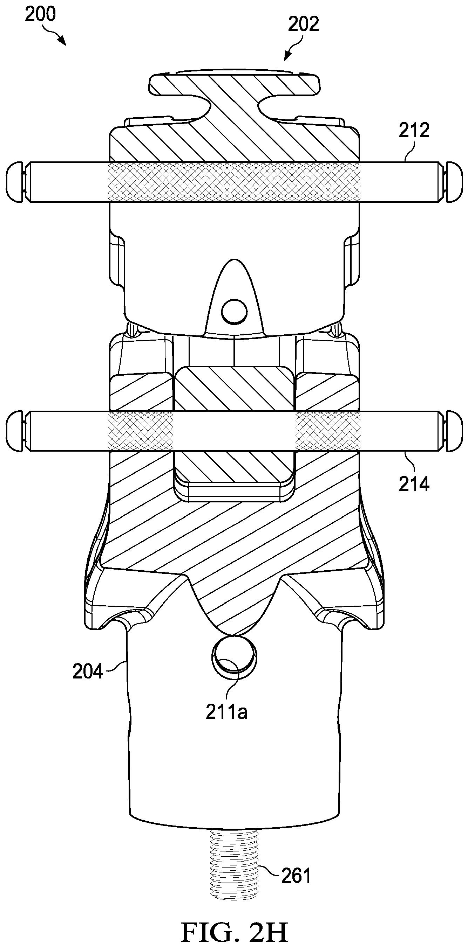

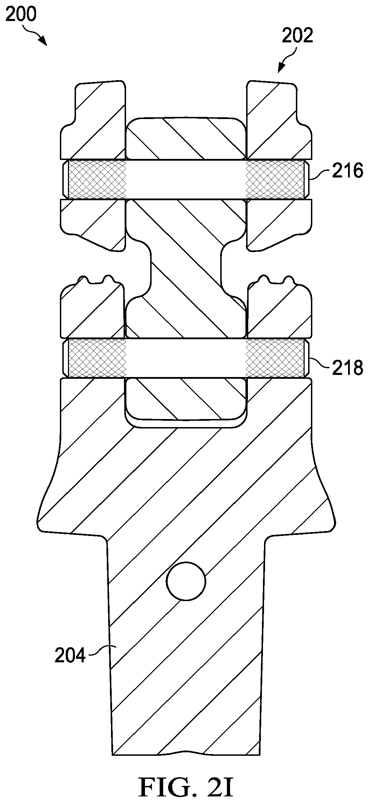

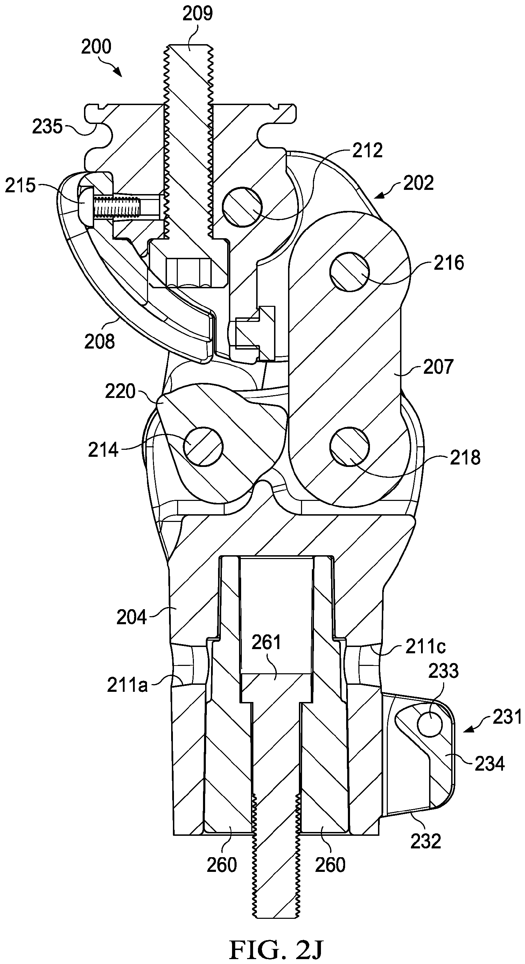

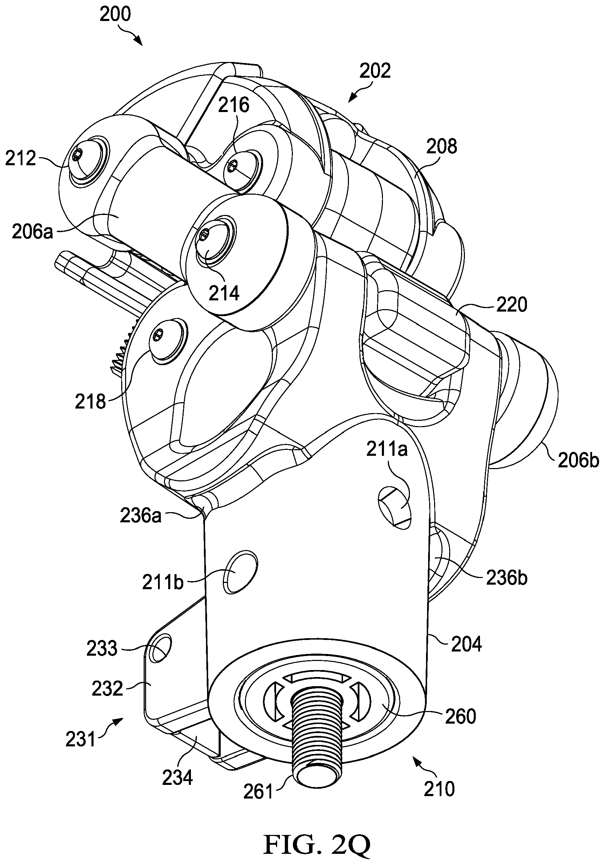

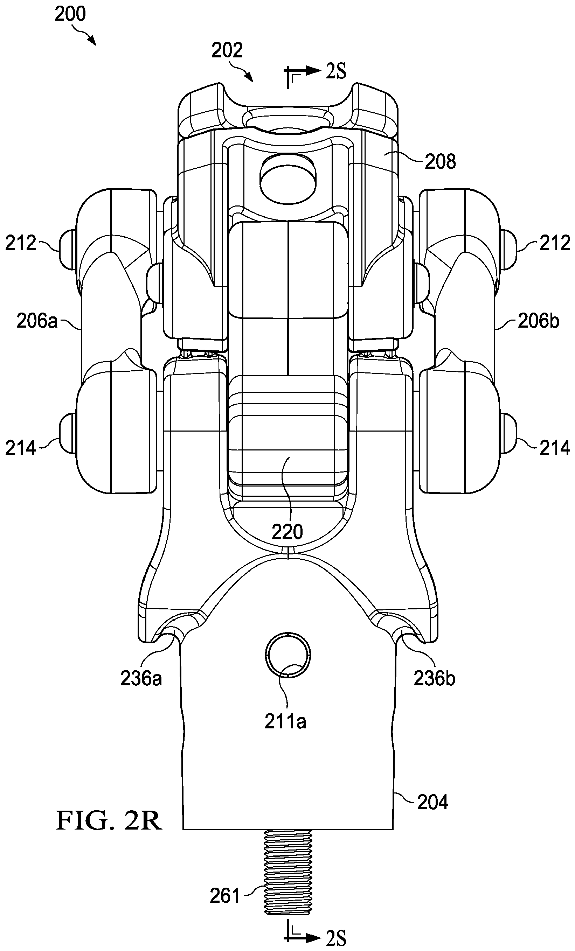

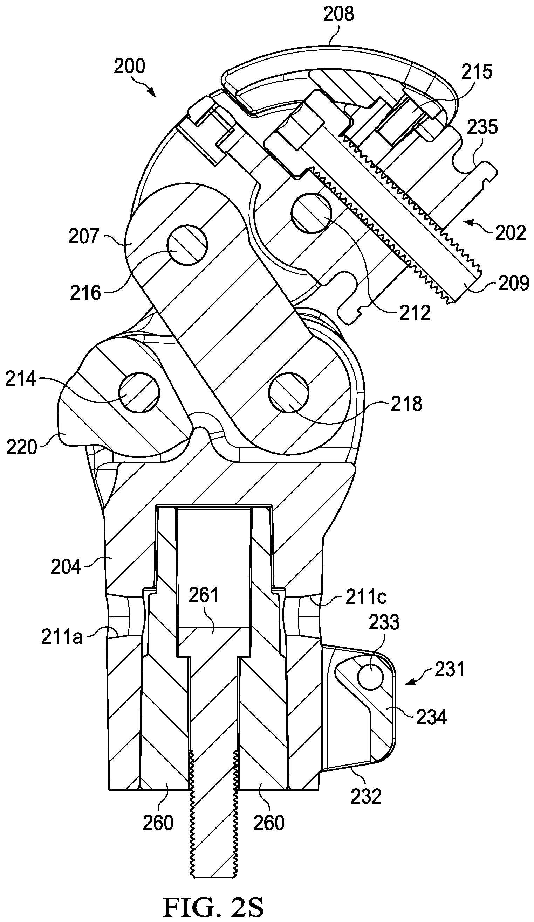

FIGS. 2A-2J show an example prosthetic knee 200 in the extended position. FIG. 2A is a perspective view. FIGS. 2B, 2C are a top and bottom view. FIGS. 2D, 2E are an anterior and posterior view. FIGS. 2F, 2G are a right and left side view. FIGS. 2H, 2I are cross-sections from an anterior view, and FIG. 2J is a cross-section view from the left side. FIGS. 2Q-2S show the example prosthetic knee 200 in the flexed position. FIG. 2Q is a perspective view, FIG. 2R is an anterior view, and FIG. 2S is a cross-section view from the left side.

The example prosthetic knee 200 includes an upper core member 202, a lower core member 204, two anterior links 206a, 206b, and a posterior link 207. In the example shown, three links are connected between the upper core member 202 and the lower core member 204. In particular, the anterior links 206a, 206b are connected to the upper core member 202 and lower core member 204 by axles 212, 214; and the posterior link 207 is connected to the upper core member 202 and lower core member 204 by axles 216, 218. A prosthetic knee can include additional links or fewer links, and the links can be connected between the core members in the manner shown or in another manner.

The example prosthetic knee 200 includes two core members 202, 204. The example core knee members transfer a load between the axles and the other components of the prosthetic knee 200. For example, the upper core member 202 transfers a load between the axles 212, 216 and a socket (or socket adapter), which may be connected (*e.g*., directly or indirectly) to the upper core member; and the lower core member 204 transfers a load between the axles 214, 218 and a pylon (or pylon adapter), which may be connected (*e.g*., directly or indirectly) to the lower core member 204. A prosthetic knee can include additional core knee members or fewer core knee members, and the core knee members can be configured as shown or in another configuration. In some example prosthetic knees, a core knee member carries a single axle, two axles, three axles, or more.

The example prosthetic knee 200 includes three links 206a, 206b, 207. The example links are elongate members that rotate with respect to the axles during the bending motion of the prosthetic knee. In the example shown, the anterior links 206a, 206b each have an upper end rotatably connected to the upper anterior axle 212 and an opposing lower end rotatably connected to the lower anterior axle 214; and the posterior link 207 has an upper end rotatably connected to the upper posterior axle 216 and a lower end rotatably connected to the lower posterior axle 218.

In some implementations, the links of the example prosthetic knee 200 are configured to transfer a load between the axles at the opposing ends of the link. For example, in some instances, the three links 206a, 206b, 207 can transfer a full external load from the upper core member 202 to the lower core member 204. In some implementations, the links of the example prosthetic knee 200 are configured to support the lower core member 204 from the upper core member 202. For example, in some instances, the three links 206a, 206b, 207 can fully support an external load carried by the lower core member 204.

The upper core member 202, lower core member 204, the links 206a, 206b, 207, and other structural components of the example prosthetic knee 200 can be made of any suitable material (*e.g*., metals, metal alloys, plastics, carbon-based materials, fiberglass, wood, etc.). In some instances, one or more of them is made of plastic material. Examples of plastic materials that can be used for the core members, the links, or other structural components include homopolymer polyacetal plastics, copolymer polyacetal plastics, other materials having similar properties, and combinations thereof. Example homopolymer polyacetal plastics include various types of DELRIN® (developed by DuPont) and generic equivalents. Example copolymer polyacetal plastics include various types of ACETRON® (developed by DuPont) and generic equivalents. Other plastic materials that can be used include various types of nylon, acrylic, and others. In some instances, one or more of the following materials may be used: nylon 6, nylon 66, polyetheretherketone (PEEK), polyetherketone (PEK), polyetherketoneketone (PEKK), polyethylene terephthalate (PET), polyimide (PI), polyarylamide, polyester liquid crystal (liquid crystal polymer, LCP), polyphthalamide (PPA), polyphenylene sulfide (PPS). Other

The upper core member 202, the lower core member 204, the links 206a, 206b, 207, and other components of the example prosthetic knee 200 can be made by any suitable manufacturing process (*e.g.*, machining, molding, casting, etc.). In some instances, one or more of them is formed by an injection molding process. In some example injection molding processes, source material is melted and forced into a mold cavity where it cools and hardens to the configuration of the cavity. The hardened material is removed from the cavity and finished. Finishing a component after the injection molding process may include, for example, annealing the material, finishing the shape of the component (*e.g.*, by cutting, machining, drilling, etc.), finishing the surface of the component (*e.g.*, by sanding, polishing, etc.), and other types of finishing processes.

The upper core member 202 of the example prosthetic knee 200 carries a bolt 209 and a replaceable knee cap 208. The example bolt 209 extends axially from the upper face of the upper core member 202. The example bolt 209 can secure the prosthetic knee 200 to a socket, such as, for example, the example socket 106 shown in FIG. 1. Additionally or alternatively, the prosthetic knee may be secured to a socket by screws, bolts, latches, clips, adapters, or other devices. In some cases, the prosthetic knee 200 can have a different type of bolt or a socket adapter (*e.g*., the socket adapter 309 shown in FIG. 3A or another type of socket adapter), which may allow the prosthetic knee 200 to couple to a particular type of socket.

The example knee cap 208 is affixed to the upper core member 202 by a removable fastener 215. The fastener 215 extends through a bore in the knee cap 208, into another bore in the upper core member 202 to secure the knee cap 208 to the upper core member 202. In the example shown, the example fastener 215 is removable and has a head that is exposed to the exterior of the prosthetic knee 200. As such, the example fastener 215 and the example knee cap 208 can both be removed without affecting operation of the prosthetic knee 200 and without requiring any other components to be disassembled or reconfigured. Other types of fasteners can be used.

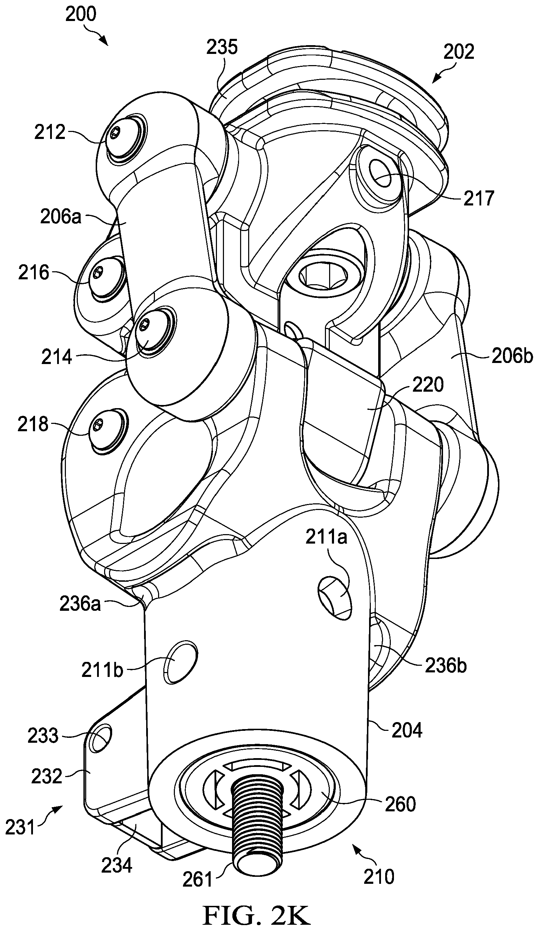

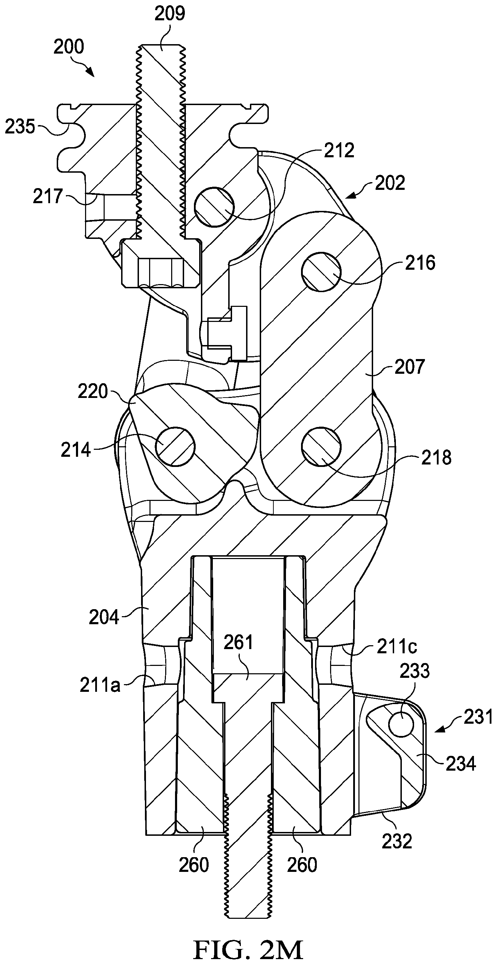

The example knee cap 208 shown in FIGS. 2A-2F and FIGS. 2M-2AF can be removed from the prosthetic knee. FIGS. 2K-2M show the example prosthetic knee 200 without the knee cap 208. In some instances, the knee cap 208 can protect the upper core member 202 from wear and damage. The example knee cap 208 shown in the figures can be removed from the upper core member 202, for example, when it becomes worn or damaged.

The knee cap 208 can provide a softer anterior surface for the prosthetic knee 200, for example, to provide cushioning when the knee cap 208 contacts the ground or another surface. In some implementations, the knee cap 208 is made of the same type of material as the core members of prosthetic knee 200. In some implementations, the knee cap 208 is made of a different, softer material than the core members of prosthetic knee 200. The knee cap 208, the core members, and the links can be made of plastic material, or they may be made of different types of material. In some instances, the knee cap 208 and other components of the knee can be made of rubber, polypropylene, wood, or other materials. In some examples, the core members and the links are made of DELRIN®, and the knee cap 208 is made of a rubber material (or another type of material) that is softer than DELRIN®.

During use, a prosthetic knee may contact the ground or another external surface. For example, many professions and hobbies require frequent kneeling, and some religious traditions involve frequent kneeling for prayer. This can potentially cause adverse wear on a prosthetic knee. A replaceable knee cap may be designed such that when a wearer kneels, the knee cap contacts the ground or other surface. Upon contacting the external surface, the softer material of the knee cap can plastically deform to provide a cushioned contact. As such, in some instances, the knee cap may protect other parts of the knee from wear or damage, or the knee cap may provide more comfortable operation for the user.

The example upper core member 202 carries an anterior axle 212 and a posterior axle 216. The anterior axle 212 in the upper core member 202 resides in a lateral bore extending the width of the upper core member 202. The anterior axle 212 is longer than the lateral width of the upper core member 202, so that sections of the axle extend from either lateral side of the upper core member 202. Each end of the axle 212 resides in a bore in one of the anterior links 206a, 206b. The anterior links 206a, 206b can rotate with respect to the upper core member 202 around the anterior axle 212. The posterior axle 216 in the upper core member 202 resides in a lateral bore extending the lateral width of the upper core member 202. The posterior axle 216 may have a length that is greater, smaller, or equal to the lateral length of the upper core member 202. The posterior axle 216 also extends through a bore in the posterior link 207. The posterior link 207 can rotate with respect to the upper core member 202 around the posterior axle 216. The axles 212, 216 may be implemented by pins, standoffs, axles, bars, bolts, or similar devices, and they may be made of metal or other suitable materials.

The lower core member 204 includes a lower bore that allows a pylon (*e.g.*, the pylon 110 in FIG. 1) to be attached to the prosthetic knee 200. Additionally or alternatively, the prosthetic knee 200 may be secured to a pylon by screws, bolts, latches, clips, adapters, or other devices. The example prosthetic knee 200 includes a pylon adapter insert 210. The pylon adapter insert 210 resides in the lower bore in the lower core knee member 204. In some cases, a pylon can be secured directly in the lower bore. The pylon adapter insert 210 allows the lower core member 204 to attach to other types of pylons, such as, for example, a pylon that would not fit into the lower bore of the lower core member 204.

The pylon adapter insert 210 includes a bolt 261 and an insert body 260. The bolt 261 extends axially from the lower core member 204. The bolt 216 can secure the pylon to the prosthetic knee 200. For example, the bolt 216 may be secured to a pylon manufactured by Endolite or another prosthetic device manufacturer. The lower core member 204 also includes four threaded bores 211a, 211b, 211c, 211d that are spaced circumferentially about the lower bore of the lower core member 204. The threaded bores 211a, 211b, 211c, 211d can receive a set screw or another type of pressure member to secure a pylon, the pylon adapter insert 210, or another object within the lower bore of the lower core member 204.

The example lower core member 204 includes an anterior axle 214 and a posterior axle 218. The anterior axle 214 in the lower core member 204 resides in a lateral bore extending the lateral width of the lower core member 204. The anterior axle 214 is longer than the lateral length of the lower core member 204, so sections of the anterior axle 214 extend from either lateral side of the lower core member 204. Each end of the anterior axle 214 resides in a bore in one of the anterior links 206a, 206b. The anterior links 206a, 206b can rotate with respect to the lower core member 204 around the anterior axle 214. The posterior axle 218 in the lower core member 204 resides in a lateral bore extending the lateral width of the lower core member 204. The posterior axle 218 may have a length that is greater, smaller, or equal to the lateral width of the lower core member 204. The posterior axle 218 also extends through a bore in the posterior link 207. The posterior link 207 can rotate with respect to the lower core member 204 about the posterior axle 218. The axles 214, 218 may be implemented by pins, standoffs, axles, bars, bolts, or similar devices, and they may be made of metal or other suitable materials.

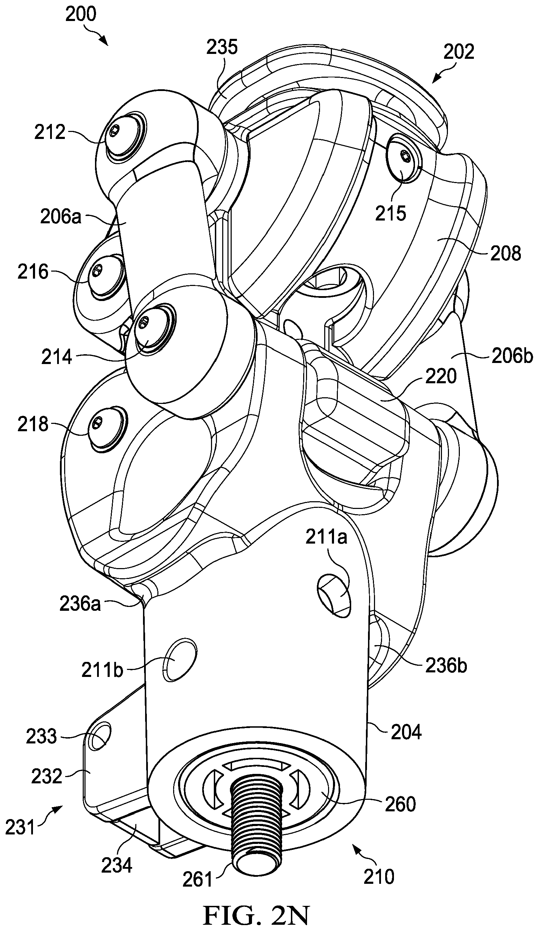

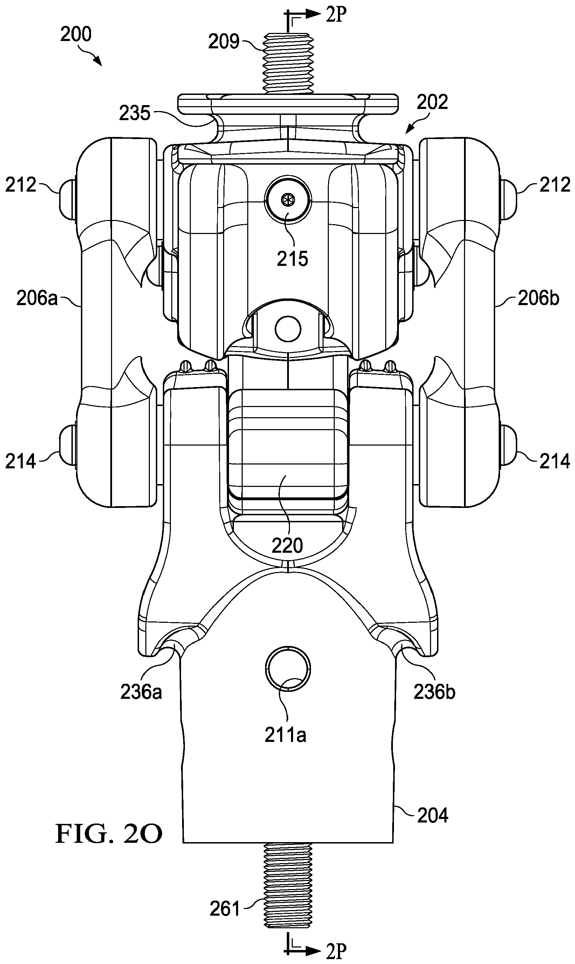

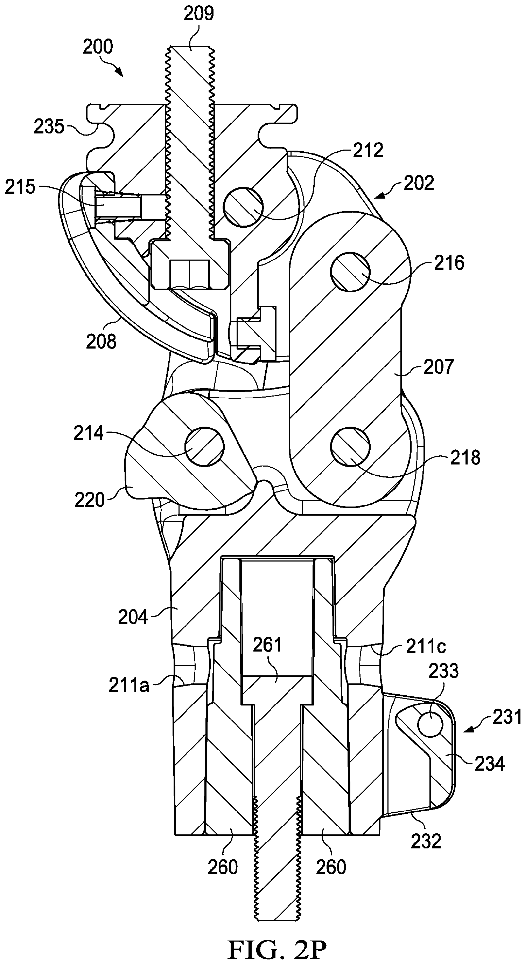

The lower core member 204 also includes a cam lock 220. FIG. 2J shows a cross-sectional view of prosthetic knee 200 extended with the cam lock 220 shown in the locked position. FIGS. 2N, 2O, 2P show the prosthetic knee 200 in the extended position with the cam lock 220 rotated to the unlocked position. In FIGS. 2Q, 2R, 2S, the prosthetic knee 200 is shown in the flexed position with the cam lock 220 rotated to the unlocked position.

The example cam lock 220 is a lobed member which can be rotated (e.g. by hand, or otherwise) between a locked lobe position and an unlocked lobe position. The cam lock 220 can be manually engaged to lock the prosthetic knee 200 in the extended position. When rotated into the locked position, a lobe on the example cam lock 220 contacts the posterior link 207, which can prevent rotation of the posterior link 207. This can prevent the prosthetic knee 200 from flexing and effectively make the prosthetic knee 200 a non-articulating knee. The user can also manually disengage the locked cam lock 220 by rotating it (*e.g.,* by hand, or otherwise) to the unlocked position, allowing the knee to flex.

The cam lock 220 may be made of metal, plastic, or other suitable material. In the example shown in FIGS. 2A-2AF, the cam lock 220 rotates around the axle 214. The same axle 214 extends through both links 206a, 206b, the lower core member 204, and the cam lock 220. The cam lock 220 is able to rotate independently of the axle 214, the lower core member 204, or the links 206a, 206b. In other implementations, the cam lock 220 may rotate around another axle. In some implementations, the cam lock 220 can be positioned on the upper core member 202 to engage the posterior link 207, or it can be positioned to engage one or more of the anterior links 206a, 206b.

The cam lock 220 can have any size or shape sufficient to prevent the prosthetic knee 200 from flexing when the cam lock 220 is rotated and engaged. In some cases, the size and shape of the lobe can be adjusted to accommodate various geometric or dynamic properties of the prosthetic knee 200. Any suitable lobed member, including the example cam lock 220 shown in the figures or another type of lobed member, can be used.

FIGS. 2H, 2I show a cross-section of prosthetic knee 200 through the anterior axles 212, 214 and posterior axles 216, 218, respectively. The shape or texture of the outer surface of the axles 212, 214, 216, 218 can be configured to prevent the axles from rotating within the core knee members. For example, the outer surfaces of the axles can be knurled along the sections that contact the inner surfaces of the respective axle bores of the core knee members. In FIGS. 2H and 2I, the parts of the axles that have a knurled texture are marked with a cross-hatching. The knurled texture can prevent the axle from rotating inside the axle bore of the core knee members. Additionally or alternatively, the axles can have other types of outer surfaces to prevent rotation of the axles within the core members. For instance, other surface textures (*e.g.,* shot peened, etc.) can be used; an adhesive material (*e.g.,* epoxy or glue) can be used; the fixed sections of the axles can have a non-cylindrical cross-sectional shape to create an interference fit; or the fixed sections of the axles can have a larger geometry to create a press fit. The sections of the axles that reside within the links can have a different type of surface (*e.g*., smooth, cylindrical, etc.) to allow rotation of the links about the axles.

FIG. 2H shows a cross-section of the example prosthetic knee 200 through the anterior axles 212, 214. The upper anterior axle 212 is knurled along a central section which resides inside the upper core member 202. The knurled texture can prevent the axle 212 from rotating with respect to the upper core member 202 during the bending motion of the prosthetic knee 200. The links 206a, 206b are mounted on the smooth sections of the axle 212, and the links can rotate around the axle 212 with respect to the upper core member 202. The lower anterior axle 214 has two separate knurled sections within the lower core member 204, which prevents the axle 214 from rotating with respect to the lower core member 204. The lower anterior axle 214 has a smooth intermediate section within the cam lock 220, which allows the cam lock 220 to rotate about the axle 214.

FIG. 2I shows a cross-section of example prosthetic knee 200 though the posterior axles 216, 218. The upper posterior axle 216 is knurled at either end where the axle 216 resides in the upper core member 202, which prevents the axle 216 from rotating with respect to the upper core member 202. The center section of the axle 216 is smooth such that the posterior link 207 can rotate about the axle 216 with respect to the upper core member 202. The lower posterior axle 218 is knurled at either end within the lower core member 204, which prevents the axle 218 from rotating in the lower core member 204. The center of the posterior axle 218 is smooth, which allows the posterior link 207 to rotate on the axle 218. Any of the axles can include a fixed portion that has a first type of outer surface (*e.g*., a knurled texture, shot-peened texture, non-cylindrical cross-section, etc.) and a rotatable portion that has a second type of outer surface (*e.g*., smooth texture, cylindrical cross-section, etc.).

The fixed portions of the axles prevent rotation of the axles within the core members, which may reduce wear on the core members over time. While the axles are fixed and static with respect to the upper and lower core members, the links can rotate to allow flexion of the prosthetic knee 200. As such, rotational wear can be focused onto the links 206a, 206b, 207, which in some cases are easier or cheaper to replace than the upper and lower core members.

Flexion of the prosthetic knee 200 causes a bending (*e.g*., extending or flexing) motion of the knee. For example, when the example prosthetic knee 200 flexes or extends, the lower core member 204 rotates with respect to the upper core member 202. In the examples shown, the lower core member 204 rotates with respect to the upper core member 202 about an instantaneous center of rotation during the bending motion. An instantaneous center of rotation for two objects can be defined based on an angle between their respective velocity vectors; or an instantaneous center of rotation can be defined based on geometric relationships among the system's rotational components. As such, a static system (*e.g.*, a system that is not in motion) can define an instantaneous center of rotation, for example, where the system is constrained to rotate along a prescribed path.

An example instantaneous center of rotation 270 is shown for an extended knee in FIG. 2AG and for a flexed knee in FIG. 2AH. The example instantaneous center of rotation 270 moves between the location shown in FIGS. 2AG and 2AH during the bending motion of the prosthetic knee 200. The location of the instantaneous center of rotation 270 at any point during the bending motion can be described in terms of the relative locations of the axles 212, 214, 216, 218.

In the examples shown, each axle 212, 214, 216, 218 defines a mechanical axis of rotation for one or more of the links with respect to a core member. Since each of the axles 212, 214, 216, 218 are parallel to each other, each pair of axles defines a plane. Plane 271 is defined by anterior axles 212, 214 and plane 272 is defined by posterior axles 216, 218. The instantaneous center of rotation 270 is located at the intersection of plane 271 and plane 272. The instantaneous center of rotation 270 is an imaginary axis parallel to the mechanical axes of rotation at axles 212, 214, 216, 218. The instantaneous center of rotation 270 can be located external to the prosthetic knee 200, as in the extended configuration shown in FIG. 2AG. The instantaneous center of rotation 270 can also be located internal to the prosthetic knee 200, as in the flexed configuration shown in FIG. 2AH.

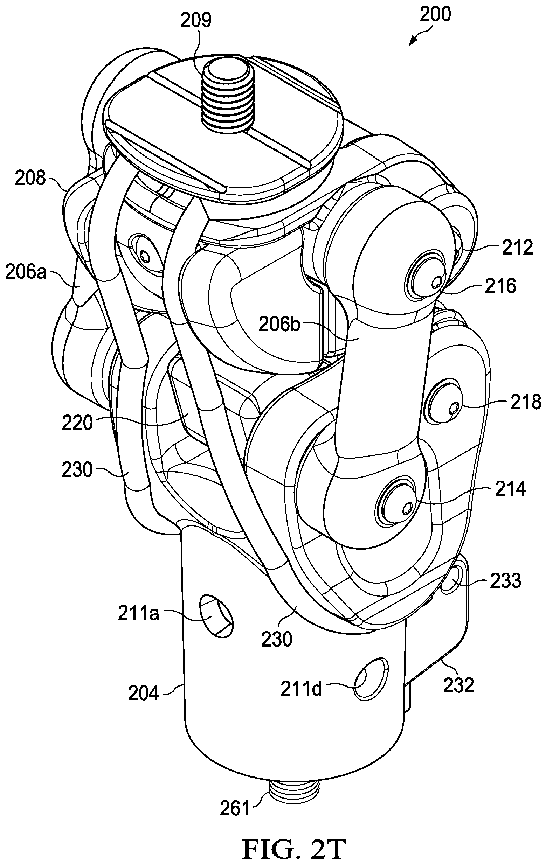

The prosthetic knee 200 can include an extension assist subsystem that biases the prosthetic knee 200 toward a particular configuration (e.g. toward the fully extended position). Generally, an extension assist mechanism can help to reduce the force the wearer uses to move the prosthetic knee toward an extended position from a flexed position. Extension assist mechanisms can be implemented in various means using different types of devices, such as springs, pistons, elastic components, or combinations of these and other components. FIGS. 2T-2AF show an example adjustable elastic cord extension assist mechanism.

As shown in the example in FIGS. 2T-2AF and 2AH, an elastic cord 230 extends between the upper core member 202 and the lower core member 204. The lower core member 204 includes an extension assist tensioner system 231 to allow adjustment of the elastic cord 230. The example extension assist tensioner system 231 includes a support 232, an axle 233, and a tensioner cam 234. The tensioner cam 234 can rotate about the axle 233 to adjust the size of an opening between the tensioner cam 234 and an opposing surface on the lower core member 204. The tensioner cam 234 can be rotated to an open position, which allows the elastic cord 230 to move through the opening with little or no resistance; and the tensioner cam 234 can be rotated to a closed position to secure the elastic cord 230 in the opening, which can provide tension on the elastic cord 230. As shown in the figures, the example prosthetic knee 200 also includes grooves that can act as guides for the elastic cord 230. The upper core member 202 includes a groove 235 around the bolt 209 at the top of upper core member 202. The lower core member 204 includes grooves 236a, 236b at the right and left sides of the lower core member 204.

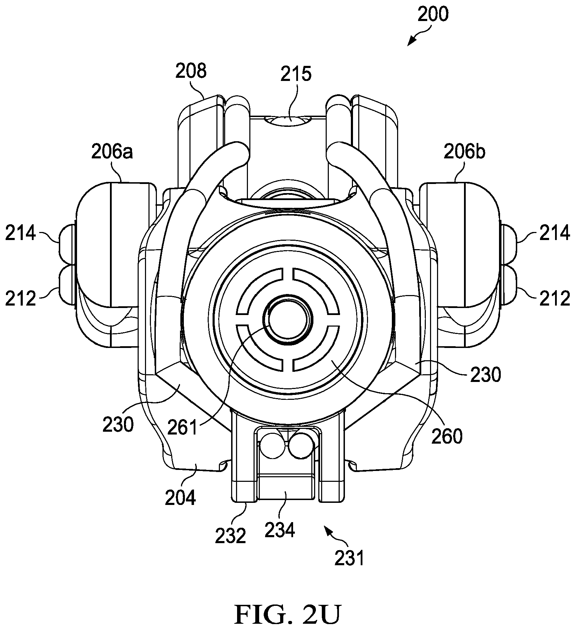

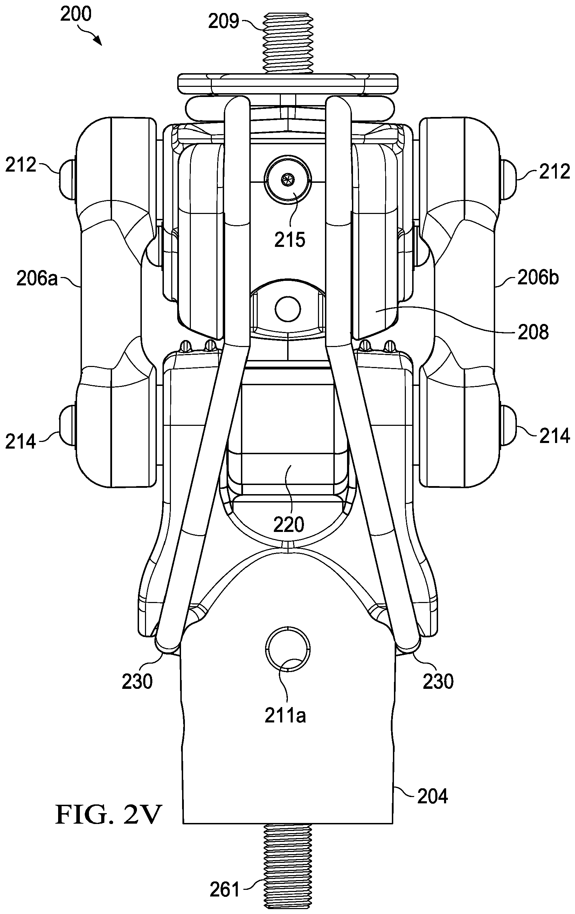

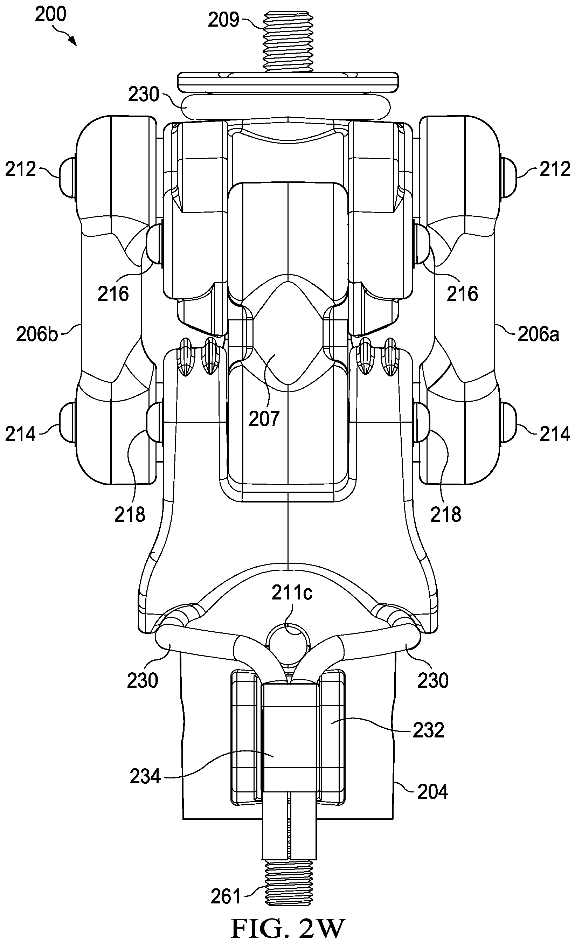

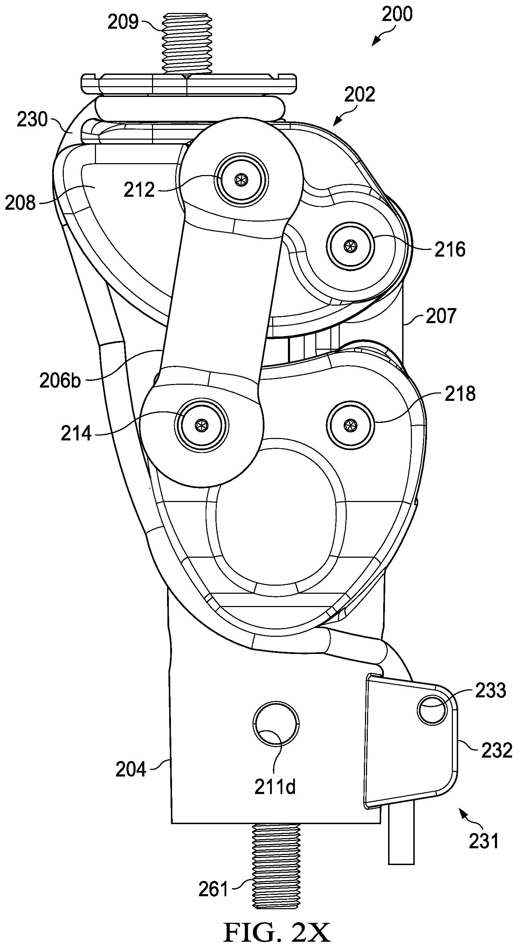

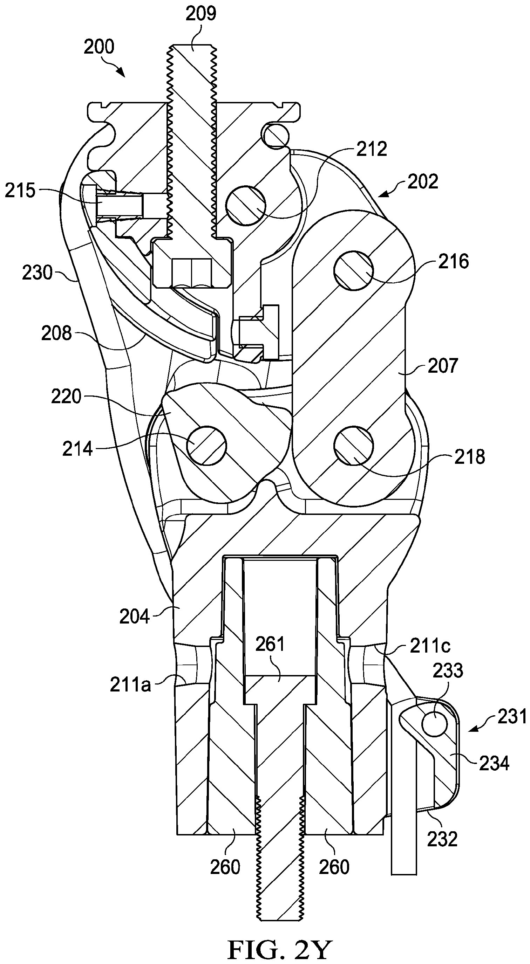

FIGS. 2T-2Y show the example prosthetic knee 200 in an extended position, with the extension assist tensioner system 231 in a locked configuration. FIG. 2T shows a perspective view; FIG. 2U is a bottom view; FIGS. 2V, 2W are anterior and posterior views; FIG. 2X is a left view; and FIG. 2Y is a cross-section of the left side view. FIGS. 2Z-2AC show the prosthetic knee 200 in a flexed position with the extension assist tensioner system 231 in the locked configuration. FIG. 2Z shows a perspective view; FIG. 2AA shows a bottom view; FIG. 2AB shows a posterior view; FIG. 2AC shows a left view. FIGS. 2AD-2AF show the prosthetic knee 200 in a flexed position with the extension assist tensioner system 231 in the unlocked configuration. FIG. 2AD shows a right view; FIG. 2AE shows a posterior view; and FIG. 2AF shows a left cross-section view.

In the example flexed position shown in FIGS. 2Z-2AF, extension assist is provided by the elastic cord 230, which extends around various components of the prosthetic knee 200 in a "C" shaped configuration. When the prosthetic knee 200 is static, the elastic cord 230 is secured at a fixed length by the extension assist tensioner system 231. In the example prosthetic knee 200, the elastic cord 230 is maintained in place by the groove 235 located on the upper core member 202 and the grooves 236a, 236b on the lower core member 204. The grooves keep the elastic cord 230 in position and prevent the elastic cord 230 from slipping or becoming dislodged. The grooves can be open to allow the elastic cord 230 to be easily removed and replaced. The grooves may be incorporated into the design of the upper and lower core members, or they may be separately attached to members or links.

In the C-type configuration, the elastic cord 230 extends from the extension assist tensioner system 231 at the back of the lower member 204, to the front of the lower core member 204 through the groove 236a. The elastic cord 230 then extends to the upper core member 202 and over the front of the knee cap 208 to the top of the upper core member 202. The elastic cord 230 loops around the upper core member 202 within the groove 235. The elastic cord 230 then extends down the front of the knee cap 208, to the front of the lower core member 204. From the front of the lower core member 204, the elastic cord 230 extends through the groove 236b, and into the extension assist tensioner system 231 at the back of the lower core member 204. The path of the elastic cord 230 may generally resemble a "C" shape when the prosthetic knee 200 is in the extended position and viewed from the side. An elastic cord can be placed in another configuration (*i.e.,* other than the C-type configuration) for extension assist.

The extension assist tensioner system 231 can maintain the elastic cord 230 at an initial length when the prosthetic knee 200 is extended. In some implementations, the initial length of the elastic cord 230 can be the slack length (*i.e.,* the length of the elastic cord 230 under no tension), or the elastic cord 230 may be maintained under some tension when the prosthetic knee 200 is extended. When the prosthetic knee 200 is flexed from the extended position, as shown in FIGS. 2Z-2AF, the elastic cord 230 is stretched from the initial length. When stretched from its initial length, the elastic cord 230 exerts a force on the upper core member 202 and the lower core member 204. The tension in the elastic cord 230 counters bending of the prosthetic knee 200 toward the flexed position, and the force exerted by stretched elastic cord 230 generally biases the prosthetic knee 200 toward the extended position. Thus, the force due to the stretched elastic cord 230 may, in some instances, reduce the amount of external force needed to return the prosthetic knee 200 from a flexed to an extended position.

FIGS. 2A-2AC show the example extension assist tensioner system 231 with the tensioner cam 234 in a locked position. FIGS. 2AD-2AF show the example extension assist tensioner system 231 with the tensioner cam 234 in the unlocked position. The tensioner support 232 extends from the lower core member 204 and supports both ends of the axle 233. The axle 233 extends through a bore in the tensioner cam 234, and the tensioner cam 234 can rotate about the axle 233.

The tensioner cam 234 is a lobed member that can rotate between a locked position and an unlocked position. The tensioner cam 234 includes a lobe and a lever. The user can rotate the tensioner cam 234 by manipulating the lever (*e.g*., by hand) to move the lobe between the locked and unlocked positions. As shown in FIG. 2Y, the lobe of the tensioner cam 234 in the locked position contacts the elastic cord 230 and applies a force that maintains tension on the elastic cord 230. As shown in FIG. 2AF, the lobe of the tensioner cam 234 in the unlocked position allows the elastic cord 230 to move, with little or no resistance, through the opening between the tensioner cam 234 and an opposing surface on the lower core member 204. In some cases, the lobe of the tensioner cam 234 may contact the elastic cord when the tensioner cam 234 is in the unlocked position. In either case, the tensioner cam 234 in the unlocked position resists movement of the elastic cord 230 through the opening less than when the tensioner cam 234 is in the locked position.

In some instances, the extension assist tensioner system 231 allows the elastic cord 230 to be temporarily removed (*e.g*., during certain types of activities) or replaced. The tensioner system 231 may also allow a wearer to adjust tension on the elastic cord 230. For example, when the tensioner cam 234 is in the unlocked position, the elastic cord 230 can be removed, repositioned, adjusted, replaced, etc. The tensioner cam 234 can then be locked, for example, to maintain the elastic cord 230 at an adjusted initial length. In some cases, adjusting the initial length of the elastic cord 230 changes (increases or decreases) the amount of biasing force applied by the elastic cord 230.

FIGS. 3A-3R show an example prosthetic knee 300 with an example pin lock system 340. FIGS. 3A-3J show the example prosthetic knee 300 extended with the example pin lock system 340 in the locked configuration. FIG. 3A shows a perspective view; FIGS. 3B and 3C show top and bottom views; FIGS. 3D and 3E are anterior and posterior views; FIGS. 3F and 3G show right and left side views. FIGS. 3H and 3I show front cross-section views through the anterior and posterior axles. FIG. 3J is a left side cross-section view.

FIGS. 3K-3O show the example prosthetic knee 300 in an extended position with the example pin lock system 340 in the unlocked configuration. FIG. 3K shows a perspective view; FIG. 3L is an anterior view; FIG. 3M is a left side cross-section view; FIG. 3N is an anterior view; FIG. 3O is a left side cross-section view. FIGS. 3L and 3M show the example prosthetic knee 300 with the knee cap 308 removed.

FIGS. 3P-3R show the prosthetic knee 300 in a flexed position with the example pin lock system 340 in the unlocked configuration. FIG. 3P is a perspective view; FIG. 3Q is a front view; FIG. 3R is a left side cross-section view.

The example prosthetic knee 300 shown in FIGS. 3A-3R is substantially similar to the example prosthetic knee 200 shown in FIGS. 2A-2AH. The example prosthetic knee 300 includes an upper core member 302, a lower core member 304, two anterior links 306a, 306b, and a posterior link 307.

In the upper core member 302, an upper anterior axle 312 resides in an anterior axle bore, and an upper posterior axle 316 resides in a posterior axle bore. In the lower core member 304, a lower anterior axle 314 resides in an anterior axle bore, and a lower posterior axle 318 resides in a posterior axle bore. Each of the anterior links 306a, 306b is rotatably secured to the upper anterior axle 312 and the lower anterior axle 314. The posterior link 307 is rotatably secured to the posterior axles 316, 318.

The upper core member 302 and the lower core member 304 can each rotate with respect to the links 306a, 306b, 307. The upper core member 302 and lower core member 304 can rotate with respect to each other about an instantaneous center of rotation. In the example prosthetic knee 300, the instantaneous center of rotation is defined by the axles 312, 314, 316, 318.

The example prosthetic knee 300 also includes a socket adapter 309, a pylon adapter insert, an extension assist tensioner system 331, and a replaceable knee cap 308. The example prosthetic knee 300 can be coupled to a socket by the socket adapter 309. The pylon adapter insert includes the bolt 361 and the insert body 360. Set screws or another type of pressure member can be installed through threaded bores 311a, 311b, 311c, 311d in the lower core member 304 to secure a pylon or a pylon adapter insert within the lower bore of the lower core member 304. The extension assist tensioner system 331 includes a tensioner cam 334, an axle 333, and supports 332. The knee cap 308 is secured to the upper core member 302 by a removable fastener 315. The fastener 315 extends through a bore in the knee cap 308, into another bore 317 in the upper core member 302 to secure the knee cap 308 to the upper core member 302.

The example extension assist tensioner system 331 can secure an elastic cord on the prosthetic knee 300 to provide extension assist for the prosthetic knee 300. The elastic cord can extend from the extension assist tensioner system 331, through the grooves 336a, 336b on the lower core member 304, and through the groove 335 on the upper core member. For example, the elastic cord can be installed in the C-type configuration of the example elastic cord 230 shown in FIGS. 2T-2AF. Or an elastic cord can be installed on the prosthetic knee 300 in another configuration.

The example prosthetic knee 300 includes an example pin lock system 340. To accommodate the pin lock system 340, the upper core member 302 and the lower core member 304 of the example prosthetic knee 300 are different from the upper core member 202 and the lower core member 204 of the example prosthetic knee 200.

The example pin lock system 340 includes a pin 342, a slot 341, an upper bore 343, and a lower bore 344. The example pin 342 is an elongate member with an end that has a generally conical shape. The example pin 342 is carried in the lower core member 304 in the lower bore 344 and the slot 341. The lower bore 344 has an opening on the upper side of lower core member 304 and extends into the lower core member 304 from the opening. Within the lower core member 304, the lower bore 344 intersects the slot 341. The pin 342 can rotate and translate vertically within the lower bore 344. In some instances, the pin 342 translates into the upper bore 343 to lock the prosthetic knee 300. The upper bore 343 extends into the upper core member 302 and has an opening on the lower side of upper core member 302. The upper bore 343, or a portion of it, can have a conical shape to accept the end of the pin 342.

When the prosthetic knee 300 is in an extended position, the lower bore 344, the pin 342, and the upper bore 343 are generally aligned with each other. The example pin lock system 340 can also include a spring in the lower bore 344, for example, to bias the pin 342 toward a locked position.

The pin lock system 340 can be adjusted between an unlocked configuration that allows flexion of the prosthetic knee 300 and a locked configuration that prevents flexion of the prosthetic knee 300. When the pin lock system 340 is in the locked position, for example, as shown in FIG. 3A, the pin 342 is positioned upward so that the top end of the pin 342 contacts the inside of the upper bore 343, while a section of the pin 342 remains inside the lower bore 344. The pin 342 extending between the upper bore 343 and the lower bore 344 can prevent the upper core member 302 from rotating with respect to lower core member 304. Thus, the example pin lock system 340 can prevent the prosthetic knee 300 from flexing and may secure the prosthetic knee 300 in the extended position.

The lower end of the pin 342 can be rotated inside the slot 341 between a locked position that prevents the pin 342 from translating vertically and an unlocked position that allows vertical translation of the pin 342. The pin lock system 340 can be unlocked by lowering the pin 342 and rotating it so that it is secured inside the slot 341, as shown in FIGS. 3K-3R. The coiled spring inside the slot 341 can apply an upward force on the pin 342 to maintain the pin 342 in the locked position until is it reset to the unlocked position. In the example prosthetic knee 300, the pin 342 is located on a lateral side of the prosthetic knee 300 such that it is accessible for adjustment by hand. The pin 342 can be located in another position.

FIGS. 3H, 3I show cross-section views of prosthetic knee 300. FIG. 3H shows a cross-section through the anterior axles 312, 314, and FIG. 3I shows a cross-section through posterior axles 316, 318. FIGS. 3H, 3I show the sections of the axles 312, 314, 316, 318 where they are knurled or textured. A section of the axle 312 is knurled or otherwise textured to prevent the axle 312 from rotating with respect to upper core member 302. Likewise, two sections of the axle 314 are knurled or otherwise textured to prevent the axle 314 from rotating with respect to lower core member 304. The axles 312, 314 are knurled where they reside inside the core members 302, 304. The axles 312, 314 are smooth elsewhere so that the associated links 306a, 306b can rotate with respect to the axles 312, 314. Similarly, axles 316, 318 are knurled in sections to prevent them from rotating with respect to upper core member 302 and lower core member 304, respectively. The axles 316, 318 are smooth in the center section, so that the link 307 can rotate about the axles 316, 318.

FIGS. 4A-4Q show another example prosthetic knee 400. FIGS. 4A-4J show the example prosthetic knee 400 extended with a knee cover 450 attached and a pin lock system 440 in the locked configuration. FIG. 4A is a perspective view, FIGS. 4B, 4C are top and bottom views, FIGS. 4D, 4E are anterior and posterior views, and FIGS. 4F, 4G are right and left views. FIGS. 4H, 4I are cross-section views through the anterior and posterior axles, respectively. FIG. 4J is a left cross-section view. FIGS. 4K-4M show the example prosthetic knee 400 extended with the knee cover 450 removed and the pin lock system 440 in the unlocked configuration. FIG. 4K is a perspective view, FIG. 4L is an anterior view, and FIG. 4M is a left cross-section view. FIG. 4N shows a perspective view of the example prosthetic knee 400 extended with the knee cover 450 attached and the pin lock system 440 in the unlocked configuration. FIGS. 4O-4Q show the example prosthetic knee 400 in a flexed position with the knee cover 450 attached. FIG. 4O is a perspective view, FIG. 4P is an anterior view, and FIG. 4Q is a left cross-section view.

The example prosthetic knee 400 shown in FIGS. 4A-4Q is substantially similar to the example prosthetic knee 200 shown in FIGS. 2A-2AH. The example prosthetic knee 400 includes an upper core member 402, a lower core member 404, two anterior links 406a, 406b, and a posterior link 407.

In the upper core member 402, an upper anterior axle 412 resides in an anterior axle bore, and an upper posterior axle 416 resides in a posterior axle bore. In the lower core member 404, a lower anterior axle 414 resides in an anterior axle bore, and a lower posterior axle 418 resides in a posterior axle bore. Each of the anterior links 406a, 406b is rotatably secured to the upper anterior axle 412 and the lower anterior axle 414. The posterior link 407 is rotatably secured to the posterior axles 316, 318.

The upper core member 402 and the lower core member 404 can each rotate with respect to the links 406a, 406b, 407. The upper core member 402 and lower core member 404 can rotate with respect to each other about an instantaneous center of rotation. In the example prosthetic knee 400, the instantaneous center of rotation is defined by the axles 412, 414, 416, 418.

The example prosthetic knee 400 also includes a socket adapter 409, a pylon adapter insert, a knee cover 450, an extension assist tensioner system 431, and a replaceable knee cap 408. The example prosthetic knee 400 can be coupled to a socket by the socket adapter 409. The pylon adapter insert includes the bolt 461 and the insert body 460. Set screws or another type of pressure member can be installed through threaded bores 411a, 411b, 411c, 411d in the lower core member 404 to secure a pylon or a pylon adapter insert within the lower bore of the lower core member 404. The extension assist tensioner system 431 includes a tensioner cam 434, an axle 433, and supports 432. The knee cap 408 is secured to the upper core member 402 by a removable fastener 415.

The example extension assist tensioner system 431 can secure an elastic cord on the prosthetic knee 400 to provide extension assist for the prosthetic knee 400. The elastic cord can extend from the extension assist tensioner system 431, through the grooves 436a, 436b on the lower core member 404, and through the groove 435 on the upper core member. For example, the elastic cord can be installed in the C-type configuration of the example elastic cord 230 shown in FIGS. 2T-2AF. Or an elastic cord can be installed on the prosthetic knee 400 in another configuration.

The example knee cover 450 is shown in FIGS. 4A-4J and FIGS. 4N-4Q. The knee cover 450 is disposed on the anterior side of the prosthetic knee 400, and has a generally curved shape. The knee cover 450 is separate from the core members 402, 404. The knee cover 450 is connected to and abuts the anterior axles 412, 414 and the anterior links 406a, 406b. The knee cover 450 includes four lateral holes which align with the ends of the anterior axles 412, 414. The knee cover 450 also includes profiled ends that grip portions of the anterior links 406a, 406b to secure the knee cover 450 to the links 406a, 406b.

During the bending motion of the prosthetic knee 400, the knee cover 450 rotates with the links 406a, 406b with respect to the upper core member 402 and lower core member 404. In other words, the rotational movement of the example knee cover 405 is identical to the rotational movement of the anterior links 406a, 406b during the bending motion of the prosthetic knee 400. The knee cap 408 rotates with the upper core member 408. As such, the knee cap 408 and the knee cover 450 rotate by different degrees during the bending motion of the knee.

When the prosthetic knee 400 is extended (as shown in FIGS. 4A-4J), the anterior face of the knee cover 450 is generally aligned with the central axis of the lower member 402, and the knee cap 408 is disposed between the upper core member 402 and the knee cover 450. When the prosthetic knee 400 is flexed (as shown in FIGS. 4N-4Q), the example knee cover 450 is angled to contact a kneeling surface, and the knee cap 408 is at least partially exposed. For example, the knee cover 450 in the flexed position would be generally disposed between the lower core member 404 and the kneeling surface, and the knee cap 408 would be generally disposed on the upper core member 402 in the forward direction.

In some instances, the knee cover 450 can protect the other components of the prosthetic knee 400. When the wearer kneels, the knee cover 450 can contact the ground and protect the prosthetic knee 400, and may provide a more comfortable support for the kneeling user. The knee cover 450 can also be removed and replaced. The curved surface of the knee cover 450 can also provide a more natural overall cosmetic appearance for the prosthetic knee 400.

The knee cover 450 can provide a softer exterior surface for the prosthetic knee 400, for example, to provide cushioning when the knee cover 450 contacts the ground or another surface. In some implementations, the knee cover 450 is made of the same type of material as the core members of prosthetic knee 400. In some implementations, the knee cover 450 is made of a different, softer material than the core members of prosthetic knee 400. The knee cover 450, the core members, and the links can be made of plastic material, or they may be made of different types of material. In some instances, the knee cover 450 and other components of the knee can be made of rubber, polypropylene, wood, or other materials. In some examples, the core members and the links are made of DELRIN®, and the knee cover 450 is made of a rubber material (or another type of material) that is softer than DELRIN®.

FIGS. 5A-5D show an example pylon adapter insert 510. FIGS. 5A and 5C are perspective views, and FIG. 5B is a side view of the example pylon adapter insert 510. FIG. 5D is a perspective view of the pylon adapter insert 510 outside the lower bore of the lower core member of an example prosthetic knee 500. The example pylon adapter insert 510 can be installed in the lower core member of a prosthetic knee, such as, for example, the pylon adapter insert 210 as shown in FIGS. 2A and 2J. Or the example pylon adapter insert 510 can be reconfigured or installed in another manner.

The example pylon adapter insert 510 includes an insert body 560 and a bolt 561. The insert body 560 is generally cylindroidal in shape. The insert body 560 includes flat regions 562a, 562b on opposing sides of the generally cylindroidal shape. The insert body 560 includes an upper opening 564 on a first axial end of the generally cylindroidal shape, and the insert body 560 includes a lower opening 563 on a second, opposing axial end of the generally cylindroidal shape. The threaded bolt 561 can be installed through the upper opening 564, and the installed threaded bolt 561 extends from the lower opening 563 as shown in FIGS. 5B and 5C.

The pylon adapter insert 510 can be configured to reside in an inner volume in the lower end of a lower core knee member. In the example shown in FIGS. 2A-2AH, set screws can be installed through the threaded bores 211a, 211b, 211c, 211d to secure the pylon adapter insert 210 in place within the inner volume of the lower core knee member. The set screws can apply pressure to the flat regions 562a, 562b of the insert body 560. The pressure from the set screws can hold the pylon adapter insert 510 firmly in place. The set screws can be removed or adjusted to allow the pylon adapter insert 510 to be removed or replaced.

The pylon adapter insert 510 allows the same prosthetic knee to be adaptable to support different types of pylons. For example, a prosthetic knee may be configured to accept the upper end of one type of pylon, and the pylon adapter insert 510 can be configured to accept the upper end of a second, different type of pylon. The threaded bolt 561, the opening 563, and other components of pylon adapter insert 510 can have a particular size or shape or configuration, for example, to accommodate a particular type of pylon fitting.

While this specification contains many specific implementation details, these should not be construed as limitations on the scope of any inventions or of what may be claimed, but rather as descriptions of features specific to particular implementations. Certain features that are described in this specification in the context of separate implementations can also be implemented in combination in a single implementation. Conversely, various features that are described in the context of a single implementation can also be implemented in multiple implementations separately or in any suitable subcombination. Moreover, although features may be described as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results.

Thus, particular implementations of the subject matter have been described. Nevertheless, various modifications may be made. Accordingly, other implementations are within the scope of the following claims.

## Claims

1. A polycentric prosthetic knee (300, 400) comprising:
a first core knee member (304, 404) that rotates with respect to a second core knee member (302, 402) about an instantaneous center of rotation during flexion of the polycentric prosthetic knee;
axles (312, 314, 316, 318, 412, 414, 416, 418) that define the instantaneous center of rotation; and
an elongate member (342) carried by the first core knee member and having an end that is translatable, through an opening in the second core knee member, between a first end position that allows the flexion and a second end position that contacts an inner surface of the second core knee member to prevent the flexion, wherein a first end of the elongate member is translatable between the first end position and the second end position, and **characterised in that** the elongate member includes a second, opposite end that is rotatable between a locked position that prevents translation of the first end and an intermediate position that allows translation of the first end from the first end position to the second end position.

2. The polycentric prosthetic knee of claim 1, wherein the elongate member is a metal pin.

3. The polycentric prosthetic knee of claim 1, comprising a spring that biases the elongate member toward the second end position.

4. The polycentric prosthetic knee of claim 1, wherein the end of the elongate member has a generally conical shape.

5. The polycentric prosthetic knee of claim 1, wherein the inner surface of the second core member has a generally conical shape to accommodate the shape of the end of the elongate member.

6. The polycentric prosthetic knee of claim 1, wherein the second end of the elongate member is hand-rotatable between the locked position and the intermediate position.

7. The polycentric prosthetic knee of claim 1, wherein the second end of the elongate member is accessible on a lateral side of the polycentric prosthetic knee.

8. The polycentric prosthetic knee of claim 1, wherein:
the second core knee member is an upper core knee member that carries an upper axis;
the first core knee member is a lower core knee member that carries a lower axis;
the polycentric prosthetic knee further comprises a link (306, 307, 406, 407) connected between the upper axis and the lower axis, the link being rotatable about the upper axis and the lower axis during a bending motion of the polycentric prosthetic knee; and
the elongate member is a pin.

9. The polycentric prosthetic knee of claim 8, wherein the pin is moveable by action of a spring carried by the lower core knee member.

10. The polycentric prosthetic knee of claim 8, wherein the pin in the first position allows the bending motion and the pin in the second position prevents the bending motion.

## Patentansprüche

1. Polyzentrische Knieprothese (300, 400), umfassend:
ein erstes Kernknieelement (304, 404), das sich in Bezug auf ein zweites Kernknieelement (302, 402) um einen Augenblicksdrehpol während der Flexion der polyzentrischen Knieprothese dreht;
Achsen (312, 314, 316, 318, 412, 414, 416, 418), die den Augenblicksdrehpol definieren; und
ein längliches Element (342), das vom ersten Kernknieelement getragen wird und ein Ende aufweist, das translatorisch verschoben werden kann durch eine Öffnung im zweiten Kernknieelement zwischen einer ersten Endposition, welche die Flexion gestattet, und einer zweiten Endposition, die mit einer Innenfläche des zweiten Kernknieelements in Kontakt steht, um die Flexion zu verhindern, wobei ein erstes Ende des länglichen Elements zwischen der ersten Endposition und der zweiten Endposition translatorisch verschoben werden kann,
und **dadurch gekennzeichnet, dass** das längliche Element ein zweites gegenüberliegendes Ende aufweist, das zwischen einer verriegelten Position, die eine translatorische Verschiebung des ersten Endes verhindert, und einer Zwischenposition, die eine translatorische Verschiebung des ersten Endes aus der ersten Endposition in die zweite Endposition gestattet, drehbar ist.

2. Polyzentrische Knieprothese nach Anspruch 1,
wobei das längliche Element ein Metallstift ist.

3. Polyzentrische Knieprothese nach Anspruch 1,
umfassend eine Feder, die das längliche Element zur zweiten Endposition vorspannt.

4. Polyzentrische Knieprothese nach Anspruch 1,
wobei das Ende des länglichen Elements eine allgemein konische Form aufweist.

5. Polyzentrische Knieprothese nach Anspruch 1,
wobei die Innenfläche des zweiten Kernelements eine allgemein konische Form aufweist, um die Form des Endes des länglichen Elements aufzunehmen.

6. Polyzentrische Knieprothese nach Anspruch 1,
wobei das zweite Ende des länglichen Elements zwischen der verriegelten Position und der Zwischenposition händisch drehbar ist.

7. Polyzentrische Knieprothese nach Anspruch 1,
wobei das zweite Ende des länglichen Elements auf einer lateralen Seite der polyzentrischen Knieprothese zugänglich ist.

8. Polyzentrische Knieprothese nach Anspruch 1, wobei:
das zweite Kernknieelement ein oberes Kernknieelement ist, das eine obere Achse trägt;
das erste Kernknieelement ein unteres Kernknieelement ist, das eine untere Achse trägt;
die polyzentrische Knieprothese ferner eine Verbindung (306, 307, 406, 407) umfasst, die zwischen der oberen Achse und der unteren Achse angeschlossen ist, wobei die Verbindung um die obere Achse und die untere Achse während einer Biegebewegung der polyzentrischen Knieprothese drehbar ist; und
das längliche Element ein Stift ist.

9. Polyzentrische Knieprothese nach Anspruch 8,
wobei der Stift durch die Wirkung einer Feder bewegbar ist, die vom unteren Kernknieelement getragen wird.

10. Polyzentrische Knieprothese nach Anspruch 8,
wobei der Stift in der ersten Position die Biegebewegung gestattet, und der Stift in der zweiten Position die Biegebewegung verhindert.

## Revendications

1. Prothèse de genou polycentrique (300, 400), comprenant:
un premier élément de genou central (304, 404) qui tourne par rapport à un second élément de genou central (302, 402) autour d'un centre de rotation instantané pendant la flexion de la prothèse de genou polycentrique;
des axes (312, 314, 316, 318, 412, 414, 416, 418) qui définissent le centre de rotation instantané, et
un élément allongé (342) porté par le premier élément de genou central et qui présente une extrémité déplaçable, à travers une ouverture dans le second élément de genou central, entre une première position d'extrémité qui permet la flexion et une seconde position d'extrémité qui est en contact avec une surface intérieure du second élément de genou central dans le but d'empêcher la flexion, dans laquelle une première extrémité de l'élément allongé est déplaçable entre la première position d'extrémité et la seconde position d'extrémité, et **caractérisée en ce que** l'élément allongé présente une seconde extrémité opposée qui est rotative entre une position verrouillée qui empêche le déplacement de la première extrémité et une position intermédiaire qui permet le déplacement de la première extrémité à partir de la première position d'extrémité jusqu'à la seconde position d'extrémité.

2. Prothèse de genou polycentrique selon la revendication 1, dans laquelle l'élément allongé est une broche métallique.

3. Prothèse de genou polycentrique selon la revendication 1, comprenant un ressort qui pousse l'élément allongé en direction de la seconde position d'extrémité.

4. Prothèse de genou polycentrique selon la revendication 1, dans laquelle l'extrémité de l'élément allongé présente une forme essentiellement conique.

5. Prothèse de genou polycentrique selon la revendication 1, dans laquelle la surface intérieure du second élément central présente une forme essentiellement conique afin de recevoir la forme de l'extrémité de l'élément allongé.

6. Prothèse de genou polycentrique selon la revendication 1, dans laquelle la seconde extrémité de l'élément allongé peut être pivotée manuellement entre la position verrouillée et la position intermédiaire.

7. Prothèse de genou polycentrique selon la revendication 1, dans laquelle la seconde extrémité de l'élément allongé est accessible par un côté latéral de la prothèse de genou polycentrique.

8. Prothèse de genou polycentrique selon la revendication 1, dans laquelle:
le second élément de genou central est un élément de genou central supérieur qui porte un axe supérieur;
le premier élément de genou central est un élément de genou central inférieur qui porte un axe inférieur;
la prothèse de genou polycentrique comprend en outre une liaison (306, 307, 406, 407) qui est connectée entre l'axe supérieur et l'axe inférieur, la liaison étant rotative autour de l'axe supérieur et de l'axe inférieur pendant un mouvement de flexion de la prothèse de genou polycentrique; et
l'élément allongé est une broche.

9. Prothèse de genou polycentrique selon la revendication 8, dans laquelle la broche est déplaçable par l'actionnement d'un ressort porté par l'élément de genou central inférieur.

10. Prothèse de genou polycentrique selon la revendication 8, dans laquelle la broche dans la première position permet le mouvement de flexion, et la broche dans la seconde position empêche le mouvement de flexion.
